(19) **Europäisches Patentamt / European Patent Office / Office européen des brevets**

(11) **EP 4 752 885 A1**

(12) **EUROPEAN PATENT APPLICATION**
published in accordance with Art. 153(4) EPC

(43) Date of publication:
03.06.2026 Bulletin 2026/23

(21) Application number: 24845961.2

(22) Date of filing: 19.07.2024

(51) International Patent Classification (IPC):
$G16B\ 30/10^{(2019.01)}$    $C12Q\ 1/6811^{(2018.01)}$
$G16B\ 50/00^{(2019.01)}$

(52) Cooperative Patent Classification (CPC):
C12Q 1/6811; G16B 30/10; G16B 50/00

(86) International application number:
PCT/KR2024/010509

(87) International publication number:
WO 2025/023659 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 25.07.2023 KR 20230096963

(71) Applicant: Seegene, Inc.
Seoul 05548 (KR)

(72) Inventors:
• JEONG, Haneul
  Seoul 08802 (KR)
• LEE, Gwangho
  Seoul 05547 (KR)

(74) Representative: Engelhard, Markus
Boehmert & Boehmert
Anwaltspartnerschaft mbB
Pettenkoferstrasse 22
80336 München (DE)

Remarks:
The complete document including Reference Table(s) and the Sequence Listing(s) can be downloaded from the EPO website

(54) **COMPUTER-IMPLEMENTED METHOD FOR PROVIDING ALIGNMENT INFORMATION ABOUT OLIGONUCLEOTIDE SEQUENCE FOR TARGET NUCLEIC ACID SEQUENCE**

(57) The present invention relates to a computer-implemented method for providing alignment information of oligonucleotides for a target nucleic acid sequence. The present invention can provide semi-global alignment of oligonucleotides for a target nucleic acid sequence by applying dynamic programming. The present invention can provide alignment information on the entire length of an oligonucleotide more accurately and at a faster rate with less computer resources than conventional methods, and can provide the coverage of an oligonucleotide for a plurality of target nucleic acid sequences.

EP 4 752 885 A1

# Fig. 1

*100*

110 — providing a target nucleic acid sequence comprising m nucleotides and an oligonucleotide sequence comprising n nucleotides

120 — performing a matrix operation of the oligonucleotide sequence for the target nucleic acid sequence to obtain a match value or mismatch value between nucleotides of the target nucleic acid sequence and the oligonucleotide sequence

130 — performing a trace operation of a n × n square matrix in the m × n matrix on which the matrix operation is performed to obtain the sum of match values and/or mismatch values of diagonal elements of the n × n square matrix

140 — when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold

**Description**

**CROSS-REFERENCE TO RELATED APPLICATIONS**

This patent application claims priority to Korean Patent Application No. 2023-0096963 filed with the Korean Intellectual Property Office on July 25, 2023, and the disclosure of the patent applications is inserted into this specification by reference.

**FIELD OF THE INVENTION**

The present invention relates to a computer-implemented method for providing alignment information of an oligonucleotide sequence for a target nucleic acid sequence.

**DESCRIPTION OF THE RELATED ART**

Polymerase Chain Reaction (PCR), a nucleic acid amplification method, includes repeated cycle processes of denaturation of double-stranded DNA, oligonucleotide primer annealing to DNA template, and primer extension by DNA polymerase (Mullis et al., U.S. Pat. Nos. 4,683,195, 4,683,202, and 4,800,159; Saiki et al., (1985) Science 230, 1350-1354).

PCR-based techniques are widely used in scientific applications or methods in biological and medical research as well as amplification of target DNA sequences, such as reverse transcriptase PCR (RT-PCR), fractional display PCR (DD-PCR), cloning of known or unknown genes by PCR, rapid amplification of cDNA terminus (RACE), random priming PCR (AP-PCR), multiplex PCR, SNP genome typing, and PCR-based genome analysis (McPherson and Moller, 2000) PCR. BIOS Scientific Publishers, Springer-Verlag New York Berlin Heidelberg, NY).

In order to provide an oligonucleotide capable of amplifying and detecting a plurality of nucleic acid sequences of a specific target nucleic acid molecule with maximum coverage, an oligonucleotide having performance capable of amplifying and/or detecting a plurality of nucleic acid sequences of a specific target nucleic acid molecule with maximum coverage should be designed, and specificity and sensitivity tests of the oligonucleotide should be performed to test the performance of the oligonucleotide.

Before the performance experiment, it was necessary to check the specificity of the designed oligonucleotide set, or after being commercialized through the performance experiment, if new nucleic acid sequences for a specific target nucleic acid molecule are sequenced or new nucleic acid sequences are registered in the database, it is necessary to additionally check the specificity of the commercialized oligonucleotide set.

Typical programs that are widely used to confirm the specificity of oligonucleotides in the related art include basic local alignment search tool (BLAST) and Primer-BLAST algorithms that can be used in national center for biotechnology information (NCBI).

In particular, as a local alignment, in the case of BLAST (blast. ncbi. nlm. nih. gov/Blast. Cgi) (Altschul et al., J. Mol. Biol. 215: 403-10 (1990)), sequence information (access number (Accession No.), etc.) including a sequence similar to an oligonucleotide sequence input as a query sequence, alignment information of a nucleic acid sequence hit with a sequence similar to a taxanomy and an oligonucleotide sequence, and the like are shown when a nucleotide BLAST is performed after inputting a nucleic acid sequence or an oligonucleotide sequence to be analyzed as a query sequence and selecting a nucleotide database including nucleic acid sequences for which similar sequences are to be found.

In addition, BLAST shows a result of a match or mismatch pattern between an oligonucleotide and an aligned nucleic acid sequence by finding a perfect match (*i.e.*, seed match) portion for each word size between the oligonucleotide and the aligned nucleic acid sequence in consideration of the word size, identity, and E-value input to the parameter, and expanding to both sides of the perfect match portion.

However, the BLAST checks whether there is a mismatch through a seed match and an extension between an oligonucleotide and an aligned nucleic acid sequence, and shows a result of a match or a mismatch pattern for some nucleotides rather than all of the oligonucleotides due to the characteristics of local alignment, and thus there is a problem in that it is impossible to know the result of the mismatch pattern for the entire length of the oligonucleotide for the nucleic acid sequence, or it is necessary to develop an additional program to know the result.

In order to solve this problem, the present applicant developed an oligonucleotide specificity evaluation (WO 2017/209575) technique to check the match or mismatch pattern results of some nucleotide parts of an oligonucleotide in which the match or mismatch pattern results are not confirmed through the BLAST. As the result, the present applicant loaded all of the plurality of nucleic acid sequences for which coverage is to be checked into a computer memory or disk to find the nucleic acid sequences previously checked through the BLAST, and found a nucleotide part showing the match or mismatch pattern results confirmed through the BLAST between the nucleic acid sequences and the aligned oligonucleotides, and then restored to some nucleotide parts of the oligonucleotide in which the match or mismatch pattern results are not confirmed through the BLAST to show the match or mismatch pattern results for the entire length of the oligonucleotide.

Using such a repair method, the conventional method for evaluating the specificity of oligonucleotides, which shows a result of a match or mismatch pattern up to some nucleotide parts of an oligonucleotide not identified through BLAST, has a problem in that it takes a long time to show a result of a match or mismatch pattern for the entire length of an oligonucleotide even when the data capacity of a plurality of nucleic acid sequences included in a nucleotide database, which is a subject of which coverage is to be checked, is adequate.

In addition, more than 13 million genomic sequences have been listed in the GISAID due to the recent global epidemic of SARS-CoV-2. When a single SARS-CoV-2 sequence is 30,000 bp long, the data capacity of the listed SARS-CoV-2 occupies more than 300Gb.

In the case that a program including the repair method is used to check the coverage of an oligonucleotide designed to detect SARS-CoV-2 with respect to SARS-CoV-2 sequences having a data capacity of 300Gb or more, and the SARS-CoV-2 sequence is loaded into a memory, the data capacity is too large to show a match or mismatch pattern result for the entire length of the oligonucleotide due to a failure of the memory, or when the SARS-CoV-2 sequence is loaded into a disk, the data capacity is too large and thus this case takes too much time in order to show a match or mismatch pattern result for the entire length of the oligonucleotide.

Accordingly, the present inventors have recognized the need to develop a technology for efficiently using computer resources and speeding up the provision of alignment information in providing alignment information of an oligonucleotide with respect to a target nucleic acid sequence in order to evaluate the specificity of the oligonucleotide or provide coverage of the oligonucleotide.

Throughout this application, various patents and publications are referenced and citations are provided in parentheses. The disclosure of these patents and publications in their entities are hereby incorporated by references into this application in order to more fully describe this invention and the state of the art to which this invention pertains.

## SUMMARY OF THE INVENTION

The present inventors have attempted to develop a method for efficiently using computer resources and speeding up the provision of alignment information in providing alignment information of oligonucleotides for a target nucleic acid sequence. As a result, the present invention has been completed by developing a technique capable of providing semi-global alignment of oligonucleotides with respect to a target nucleic acid sequence by applying dynamic programming, and confirming that alignment information with respect to the entire length of the oligonucleotides can be provided more accurately and rapidly with less computer resources than the conventional method.

Accordingly, it is an object of the present invention to provide a computer-implemented method for providing alignment information of an oligonucleotide for a target nucleic acid sequence.

It is another object of the present invention to provide a computer readable storage medium containing instructions to configure a processor to perform a method for providing alignment information of an oligonucleotide for a target nucleic acid sequence.

Other objects and advantages of the present invention will become apparent from the detailed description to follow taken in conjugation with the appended claims and drawings.

## DETAILED DESCRIPTION OF THIS INVETNION

### I. Method of providing alignment information without considering a gap (first aspect)

In an aspect of the present invention, there is provided a computer-implemented method for providing alignment information of an oligonucleotide for a target nucleic acid sequence comprising:

(a) providing a target nucleic acid sequence comprising m nucleotides and an oligonucleotide sequence comprising n nucleotides; wherein the target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type,

(b) performing a matrix operation of the oligonucleotide sequence for the target nucleic acid sequence to obtain a match value or mismatch value between nucleotides of the target nucleic acid sequence and the oligonucleotide sequence; wherein the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score,

(c) performing a trace operation of a n × n square matrix in the m × n matrix on which the matrix operation is performed to obtain the sum of match values and/or mismatch values of diagonal elements of the n × n square matrix; and

(d) when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold value; wherein the alignment information comprises information selected from the group consisting of a match and/or mismatch between nucleotides of the target nucleic acid sequence and the oligonucleotide sequence, an alignment pattern comprising the match and/or mismatch information, the number of the mismatch, and the position of the oligonucleotide sequence in aligned target nucleic acid sequences.

The present inventors have attempted to develop a method for efficiently using computer resources and speeding up the provision of alignment information in providing alignment information of oligonucleotides for a target nucleic acid sequence. As a result, according to the present invention, that alignment information with respect to the entire length of the oligonucleotides can be provided more accurately and rapidly with less computer resources than the conventional method is confirmed by developing a technique capable of providing semi-global alignment of oligonucleotides with respect to a target nucleic acid sequence by applying dynamic programming.

The method of the first aspect of the present invention is directed to a technique for providing alignment information of an oligonucleotide relative to a target nucleic acid sequence without considering gaps.

When the target nucleic acid sequence and the oligonucleotide sequence are aligned, a gap is formed in the target nucleic acid sequence and/or the oligonucleotide sequence in order to show an alignment result showing a predetermined similarity. However, when the oligonucleotide hybridizes to the target nucleic acid sequence present in the nucleic acid sample, no gap is formed. Accordingly, in consideration of hybridization to an actual target nucleic acid sequence, there is a need to provide alignment information without a gap between the target nucleic acid sequence and the oligonucleotide sequence, and thus the method of the first aspect of the present invention is intended to provide alignment information without considering a gap.

As used herein, "alignment information that does not consider a gap" refers to alignment information without a gap.

"Dynamic programming" used in the present specification is a technique of optimization theory, and is an algorithm design technique for efficiently obtaining a value by using a value in a different range to obtain a value up to a specific range. In other words, dynamic programming collectively refers to an algorithm that considers a problem as an extension of a smaller problem and uses a solution obtained in the past to solve the problem.

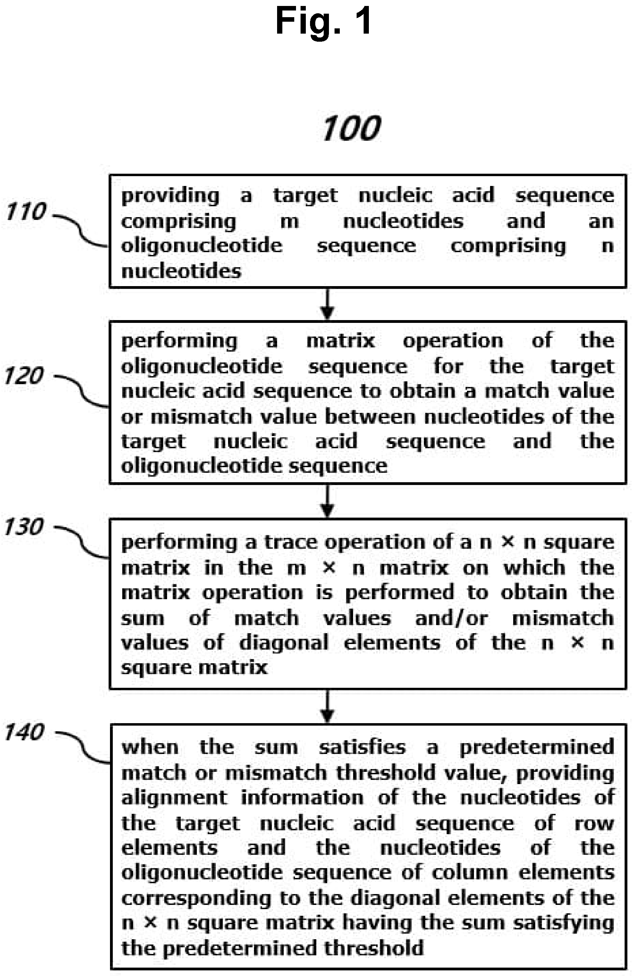

FIG. 1 is a flowchart of processes of performing a method of the present invention according to an embodiment of the present invention. The method of the present invention is described with reference to FIG. 1 as follows:

Step (a): providing a target nucleic acid sequence and an oligonucleotide sequence (110)

First, the method of the present invention comprises (a) providing a target nucleic acid sequence comprising m nucleotides and an oligonucleotide sequence comprising n nucleotides. The target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type.

The method of the present invention is a computer-implemented method, and the provision of the target nucleic acid sequence and the oligonucleotide sequence is performed by inputting information of the target nucleic acid sequence and the oligonucleotide sequence into a User Interface (UI).

Although it is described herein that m nucleotides of a target nucleic acid sequence are provided as a m×1 matrix and n nucleotides of an oligonucleotide sequence are provided as a 1×n matrix, it is clearly understood by those skilled in the art that m nucleotides of a target nucleic acid sequence are provided as a 1×m matrix and n nucleotides of an oligonucleotide sequence are provided as a n×1 matrix, thereby performing the method of the present invention described below. It is also clearly understood by those skilled in the art that methods of the present invention performed by providing m nucleotides of a target nucleic acid sequence as a 1×m matrix and n nucleotides of an oligonucleotide sequence as a n×1 matrix are also included in the scope of the present invention.

That is, it may be clearly understood by those skilled in the art that the method of the present invention performed by providing m nucleotides of a target nucleic acid sequence as a m×1 matrix and providing n nucleotides of an oligonucleotide sequence as a 1×n matrix, and the method of the present invention performed by providing m nucleotides of a target nucleic acid sequence as a 1×m matrix and providing n nucleotides of an oligonucleotide sequence as a n×1 matrix, are considered equivalent under the doctrine of equivalents.

When the order of the rows and columns of the target nucleic acid sequence and the oligonucleotide sequence is changed, the order is changed and performed in a process to be described later.

As used herein, the term "target nucleic acid sequence" or "target sequence" refers to a specific nucleic acid sequence including a specific target nucleic acid molecule of a specific organism to be amplified and/or detected by an oligonucleotide. As used herein, the term "target nucleic acid sequence" or "target sequence" refers to a specific nucleic acid sequence including a specific target nucleic acid molecule of a specific organism for which alignment information of an oligonucleotide is to be identified.

As used herein, the term "organism" means an organism belonging to one genus, species, subspecies, subtype, ginotype, sirotype, strain, isolate, or cultivar. The organism includes, for example, prokaryotic cells (*e.g., Mycoplasma pneumoniae, Chlamydophila pneumoniae, Legionella pneumophila, Haemophilus influenzae, Streptococcus pneumoniae, Bordetella pertussis, Bordetella parapertussis, Neisseria meningitidis, Listeria monocytogenes, Streptococcus agalactiae, Campylobacter, Clostridium difficile, Clostridium perfringens, Salmonella, Escherichia coli, Shigella, Vibrio, Yersinia enterocolitica, Aeromonas, Chlamydia trachomatis, Neisseria gonorrhoeae, Trichomonas vaginalis, Mycoplasma hominis, Mycoplasma genitalium, Ureaplasma urealyticum, Ureaplasma parvum, Mycobacterium tuberculosis*), eukaryotic cells (*e.g.,* protozoa and parasites, fungi, yeasts, higher plants, lower animals and higher animals including mammals and humans), viruses, or viroids. Examples of parasites in the eukaryotic cells include *Giardia lamblia, Entamoeba histolytica, Cryptosporidium, Blastocystis hominis, Dientamoeba fragilis,* and *Cyclospora cayetanensis.* Examples of such viruses which cause respiratory disease, include influenza A virus (Flu A), influenza B virus (Flu B), respiratory syncytial virus A (RSV A), respiratory syncytial virus B (RSV B), parainfluenza virus 1 (PIV 1), parainfluenza virus 2 (PIV 2), parainfluenza virus 3 (PIV 3), parainfluenza virus 4 (PIV 4), metapneumovirus (MPV), human enterovirus (HEV), human bocavirus (HBoV), human rhinovirus (HRV), coronavirus (*e.g.,* CoV NL63, CoV 229E, CoV OC43, CoV HKU1, SARS-CoV, MERS-CoV, SARS-CoV-2) and adenovirus, and more specifically SARS-CoV-2. Examples of such viruses include noroviruses, rotaviruses, adenoviruses, astroviruses, and sapoviruses, which cause gastrointestinal diseases. In addition, examples of the virus include human papillomavirus (HPV), middle east respiratory syndrome-related coronavirus (MERS-CoV), dengue virus, herpes simplex virus (HSV), human herpes virus (HHV), epstein-Barr virus (EMV), varicella zoster virus (VZV), Cytomegalovirus (CMV), HIV, hepatitis virus, and polyovirus.

As used herein, the term "target nucleic acid molecule", "target molecule", or "target nucleic acid" refers to a nucleotide molecule in an organism to be detected. Target nucleic acid molecules are generally given specific names and include the whole genome and all nucleotide molecules constituting the genome (*e.g.,* genes, pseudogenes, non-coding sequence molecules, untranslated regions, and partial regions of the genome). The target nucleic acid molecule includes, for example, a nucleic acid of an organism.

In the present specification, the length of the sequence may be expressed using a nucleotide, a base, bp, or mer, and the length of the sequence may be expressed through the number of nucleotides.

Although it is difficult to specify because the length of the target nucleic acid sequence may vary, for example, when the target nucleic acid sequence is a SARS-CoV-2 sequence and its length is 30,000 bp, the target nucleic acid sequence provided in this step may be expressed as including 30,000 nucleotides.

According to an embodiment of the present invention, the m is a natural number. The m may vary depending on the length of the target nucleic acid sequence. Specifically, the m may be selected from 10 to 50,000. In addition, the m is at least 30, at least 50, at least 100, at least 200, at least 300, at least 400, at least 500, at least 600, at least 700, at least 800, at least 900, or at least 1000.

As used herein, the term "oligonucleotide" refers to a short polynucleotide whose alignment information (or coverage) is to be confirmed. The oligonucleotide or oligonucleotide sequence may be referred to as "query" or "query sequence".

As used herein, the term "oligonucleotide" refers to a natural or modified monomer or linear oligomer of linkages, includes deoxyribonucleotides and ribonucleotides, can be specifically hybridized to a target nucleic acid sequence, and is naturally present or artificially synthesized. Oligonucleotides are particularly single chain or single strand for maximum efficiency in hybridization. Specifically, the oligonucleotide is an oligodeoxyribonucleotide. Oligonucleotides used in the present invention may include naturally occurring dNMPs (*i.e.,* dAMP, dGMP, dCMP, and dTMP), nucleotide analogs, or derivatives. In addition, the oligonucleotide may also include ribonucleotides. For example, the oligonucleotides used in the present invention are backbone modified nucleotides such as Peptide Nucleic Acid (PNA) (M. Egholm et al., Nature, 365:566-568 (1993)), Locked Nucleic Acid (LNA) (WO1999/014226), Bridged Nucleic Acid (BNA) (WO2005/021570), phosphorothioate DNA, phosphorodithioate DNA, phosphoroamidate DNA, amide-linked DNA, MMI-linked DNA, 2'-O-methyl RNA, alpha-DNA and methylphosphonate DNA, sugar modified nucleotides such as 2'-O-methyl RNA, 2'-fluoro RNA, 2'-amino RNA, 2'-O-alkyl DNA, 2'-O-allyl DNA, 2'-O-alkynyl DNA, hexose DNA, pyranosyl RNA, and anhydrohexitol DNA, and base-modified nucleotides, such as C-5 substituted pyrimidine (the substituent including fluoro-, bromo-, chloro-, iodo-, methyl-, ethyl-, vinyl-, formyl-, ethynyl-, propynyl-, alkynyl-, thiazolyl-, imidazolyl-, and pyridyl-), 7-deazapurines with C-7 substituent (substituent including fluoro-, bromo-, chloro-, iodo-, methyl-, ethyl-, vinyl-, formyl-, alkynyl-, alkenyl-, thiazolyl-, imidazolyl-, and pyridyl-), inosine, and diaminopurine. Especially, as used herein, the term "oligonucleotide" is a single strand composed of deoxyribonucleotides. The term "oligonucleotide" includes oligonucleotides that hybridize with cleavage fragments occurring depending on a target nucleic acid sequence.

According to an embodiment of the present invention, the oligonucleotide is a primer or a probe.

As used herein, the term "primer" refers to an oligonucleotide that can act as a point of initiation of synthesis when placed under conditions in which synthesis of primer extension products complementary to a target nucleic acid strand (a template) is induced, *i.e.,* in the presence of nucleotides and a polymerase, such as DNA polymerase, and appropriate temperature and pH conditions. The primer needs to be long enough to prime the synthesis of extension products in the presence of a polymerase. The appropriate length of the primer is determined according to a plurality of factors, including temperatures, fields of application, and primer sources.

As used herein, the term "probe" refers to a single-stranded nucleic acid molecule containing a portion or portions that are complementary to a target nucleic acid sequence. The probe may also contain a label capable of generating a signal for target detection.

The oligonucleotides used in the present invention are, for example, 10-60 nucleotides, 10-50 nucleotides, 10-45 nucleotides, 10-40 nucleotides, 10-35 nucleotides, 15-60 nucleotides, 15-50 nucleotides, 15-45 nucleotides, 15-40 nucleotides, 15-35 nucleotides, 20-60 nucleotides, 20-50 nucleotides, 20-45 nucleotides, 20-40 nucleotides, or 20-35 nucleotides in length.

When the length of the oligonucleotide is 60 nucleotides, it may be expressed as the oligonucleotide comprises 60 nucleotides.

According to an embodiment, the n is a natural number. Specifically, the n may be selected from 10 to 60.

According to an embodiment of the present invention, the m has a number greater than n.

As used herein, the term "annealing" or "priming" refers to the juxtaposition of an oligodeoxynucleotide or nucleic acid to a template nucleic acid, the juxtaposition causes polymerase to polymerize the nucleotide to form a nucleic acid molecule complementary to the template nucleic acid or a portion thereof. As used herein, the term "hybridization" refers to forming a double chain nucleic acid from a complementary single chain nucleic acid. The terms "annealing" and "hybridization" are not different and are used interchangeably herein.

The oligonucleotide may have typical primer and probe structure composed of a sequence hybridizing with a target nucleic acid sequence. Alternatively, the oligonucleotides may have distinctive structures through structural modification thereof. For example, the oligonucleotides may have structures of Scorpion primer, Molecular beacon probe, Sunrise primer, HyBeacon probe, tagging probe, DPO primer or probe (WO 2006/095981), and PTO probe (WO 2012/096523).

The oligonucleotide may be modified oligonucleotide, such as a degenerate base-containing oligonucleotide and/or a universal base-containing oligonucleotide, in which degenerate bases and/or universal bases are introduced into a conventional primer or probe. As used herein, the terms "conventional primer", "conventional probe", and "conventional oligonucleotide" refer to a typical primer, probe, and oligonucleotide, into which a degenerate base or non-natural base is not introduced. According to an embodiment of the present invention, in the degenerate base-containing oligonucleotide or universal base-containing oligonucleotide, at least 50%, at least 60%, at least 70%, at least 80%, at least 90%, or at least 95% are non-modified oligonucleotides. According to an embodiment of the present invention, the number of degenerate bases or universal bases introduced into the conventional oligonucleotide is in the range of specifically 7 or fewer, 5 or fewer, 4 or fewer, 3 or fewer, or 2 or fewer. Alternatively, the proportion of use of the degenerate bases and/or universal bases introduced into the conventional oligonucleotide is specifically 25% or less, 20% or less, 18% or less, 16% or less, 14% or less, 12% or less, 10% or less, 8% or less, or 6% or less. The proportion of use of the degenerate bases or universal bases represents a proportion of the degenerate bases or universal bases relative to a total of the nucleotides of the oligonucleotide into which the degenerate bases or universal bases are introduced.

The degenerate bases include a variety of degenerate bases known in the art as follows: R: A or G; Y: C or T; S: G or C; W: A or T; K: G or T; M: A or C; B: C, G or T; D: A, G or T; H: A, C or T; V: A, C or G; N: A, C, G or T.

The universal bases include a variety of universal bases known in the art as follows: deoxyinosine, inosine, 7-deaza-2'-deoxyinosine, 2-aza-2'-deoxyinosine, 2'-OMe inosine, 2'-F inosine, deoxy 3-nitropyrrole, 3-nitropyrrole, 2'-OMe 3-nitropyrrole, 2'-F 3-nitropyrrole, 1-(2'-deoxy-beta-D-ribofuranosyl)-3-nitropyrrole, deoxy 5-nitropyrrole, 5-nitroindole, 2'-OMe 5-nitroindole, 2'-F 5-nitroindole, deoxy 4-nitrobenzimidazole, 4-nitrobenzimidazole, deoxy 4-aminobenzimidazole, 4-aminobenzimidazole, deoxy nebularine, 2'-F nebularine, 2'-F 4-nitrobenzimidazole, PNA-5-introindole, PNA-nebularine, PNA-inosine, PNA-4-nitrobenzimidazole, PNA-3-nitropyrrole, morpholino-5-nitroindole, morpholino-nebularine, morpholino-inosine, morpholino-4-nitrobenzimidazole, morpholino-3-nitropyrrole, phosphoramidate-5-nitroindole, phosphoramidate-nebularine, phosphoramidate-inosine, phosphoramidate-4-nitrobenzimidazole, phosphoramidate-3-nitropyrrole, 2'-O-methoxyethyl inosine, 2'-O-methoxyethyl nebularine, 2'-O-methoxyethyl 5-nitroindole, 2'-O-methoxyethyl 4-nitrobenzimidazole, 2'-O-methoxyethyl 3-nitropyrrole, and a combination thereof. More specifically, the universal base is deoxyinosine, inosine, or a combination thereof.

According to an embodiment of the present invention, the oligonucleotide is an oligonucleotide represented by the following formula (I):

5'-X-Y-Z-3'          (I)

wherein, X represents a portion comprising a hybridizing nucleotide sequence that is hybridized with the target nucleic acid sequence, Y represents a separation portion comprising two or more consecutive bases that do not involve in Watson-Crick base pairing, and Z represents a portion comprising a hybridizing nucleotide sequence that is hybridized with the target nucleic acid sequence.

The oligonucleotide of formula (I) has three different portions with distinct properties, and its annealing specificity for the target nucleic acid sequence is determined in duplicate by its two separate portions, portion X and portion Z.

In general, the annealing specificity of a conventional (typical) primer or probe is governed by its overall sequence. In contrast, the annealing specificity of the oligonucleotide of formula (I) is determined in duplicate by two portions separated by portion Y, *i.e.,* portion X and portion Z.

In the oligonucleotide of formula (I), portion Y comprises two or more consecutive bases, each of which is not involved in a Watson-Crick base pair.

In the present specification, a Watson-Crick base pair or pairing means that adenine (A) binds to thymine (T) or uracil (U), while guanine (G) binds to cytosine (C).

Thus, a base that is not involved in a Watson-Crick base pair or pairing refers to any base that does not form a Watson-Crick base pair with a base that is opposite within the target nucleic acid sequence. In particular, bases that are not involved in the Watson-Crick base pair include any base that exhibits a lower strength (low melting temperature) of base pair formation between the base and the opposite base within the target nucleic acid sequence than the strength of base pair formation between the natural bases.

In one embodiment, portion Y is designed to have the lowest Tm value of the three portions when the oligonucleotide is annealed to the target nucleic acid sequence.

These bases, which are not involved in the Watson-Crick base pair, create a bubble structure during annealing (hybridization) or amplification, particularly under conditions in which portion X and/or Y specifically anneal (hybridize) to the target nucleic acid sequence, to divide portion X and portion Z, thereby improving the anneal specificity of the primer or probe to the target nucleic acid sequence.

Examples of bases not involved in the Watson-Crick base pair include: (i) an unnatural base; (ii) a universal base; and (iii) a mismatched base. In one embodiment, the base included in a separation portion Y is selected from an unnatural base; a universal base; a mismatched base and combinations thereof.

As used herein, the term "unnatural base or non-natural base" refers to a derivative of a natural base such as adenine (A), guanine (G), thymine (T), cytosine (C), and uracil (U), which can form hydrogen-bonded base pairs with each other (see U.S. Pat. No. 8,440,406).

The non-natural base included in the oligonucleotide of Formula (I) is not involved in the Watson-Crick base pair when the opposite base in the target nucleic acid sequence is a natural base. Base pairing formation between the non-natural base and the opposite base in the target nucleic acid sequence has a lower strength (low melting temperature) compared to base pairing formation between natural bases. Thus, this base pair formation is responsible for creating bubble structures and isolating portions X and Z.

Specific examples of non-natural bases include the following base pairs combined: iso-C/iso-G, iso-dC/iso-dG, K/X, H/J, and M/N (see U.S. Pat. Nos. 7,422,850 and 8,440,406).

As used herein, the term "universal base" refers to a base capable of forming a base pair without distinction from each of natural DNA/RNA bases, and the base pair is not involved in a Watson-Crick base pair.

Base pairing formation between the universal base included in the oligonucleotide of Formula (I) and the opposite base included in the target nucleic acid sequence has a lower strength (low melting temperature) compared to base pairing formation between natural bases. Examples of the universal base are as described above.

As used herein, the term "mismatched base" refers to a base that cannot form a hydrogen bond base pair with an opposite base in a target nucleic acid sequence (see WO 2013/123552 and WO 2014/124290). The mismatched base may vary depending on the type of opposite base in the target nucleic acid.

Since the mismatched base contained in the oligonucleotide of formula (I) cannot form a base pair with the opposite base contained within the target nucleic acid, portion Y containing the mismatched base serves to create a bubble structure and separate portions X and Z.

Portion Y may have two consecutive bases that are not involved in the Watson-Crick base pair, preferably 3, 4, 5, 6, 7, or more consecutive bases that are not involved in the Watson-Crick base pair.

In the oligonucleotide of formula (I), portions X and Z are each a portion with a hybridization nucleotide to the target nucleic acid sequence, *i.e.,* a portion with a hybridization nucleotide sequence complementary to a location on the template nucleic acid that is each hybridized.

The term "complementary" is used herein to mean that it is sufficiently complementary to selectively hybridize to a target nucleic acid sequence under a designated annealing condition or strict condition, and includes the terms "substantially complementary" and "fully complementary", preferably fully complementary.

The lengths of portion X and portion Z can range from 3 to 50 nucleotide residues, respectively.

In one embodiment, portion X is longer than portion Z. Specifically, the length of portion X is 15 to 50, 15 to 40, 15 to 30, or 15 to 25 nucleotide residues.

The length of the Z portion is from 3 to 15, from 3 to 12, or from 3 to 10 nucleotide residues.

In another embodiment, portion Z is longer than portion X. Specifically, the length of portion Z is 15 to 50, 15 to 40, 15 to 30, or 15 to 25 nucleotide residues. The length of portion X is 3 to 15, 3 to 12, or 3 to 10 nucleotide residues.

In an embodiment, Tm of each of portions X and Z ranges from 6°C to 80°C, from 6°C to 70°C, from 6°C to 50°C, from 6°C to 40°C, from 10°C to 80°C, from 10°C to 70°C, from 10°C to 60°C, from 10°C to 50°C, from 10°C to 40°C, from 20°C to 80°C, from 20°C to 70°C, from 20°C to 60°C, from 20°C to 50°C, from 20°C to 40°C, from 30°C to 80°C, from 30°C to 70°C, from 30°C to 60°C, from 30°C to 50°C, or 30°C 40°C. In an embodiment, the Tm of portion Y is 1°C to 15°C, 1°C to 20°C, 1°C to 5°C, 2°C to 15°C, 2°C to 10°C, 2°C to 5°C, 3°C to 15°C, 3°C to 10°C, or 3°C to 5°C. In one embodiment, the Tm of portion Y is lower than the Tm of each of portion X and Z.

In one embodiment, the Tm of portion X is higher than the Tm of portion Z. In certain embodiments, the Tm of portion X is 5°C, 10°C, 15°C, 20°C or 25°C higher than the Tm of portion Z. In another embodiment, the Tm of portion Z is higher than the Tm of portion X. In certain embodiments, the Tm of portion Z is higher than the Tm of portion Z by 5°C, 10°C, 15°C, 20°C or 25°C.

In the oligonucleotide of formula (I), either or both of the X and Z portions may comprise at least one universal base or degenerate base.

In an embodiment, when any one or both of portion X and portion Z in the oligonucleotide of formula (I) comprise two or more universal bases, the universal bases are not continuously present in the oligonucleotide sequence, but are present separately. If the Y portion also contains two or more consecutive universal bases, then the two or more universal bases contained in either or both of the X portion and the Z portion are distinguished from the two or more consecutive universal bases in the Y portion in that they exist separately in the sequence.

As used herein, the term "degenerate base" means that any one of four bases (A, C, G, or T) or a specific subset of four bases (2 or 3 bases) may be present at a designated nucleotide position. In addition, the term refers to the possibility of two or more bases at a specific position. One oligonucleotide sequence can be synthesized to have multiple bases in the same position, which is often referred to as a degenerate base referred to as a "wobble" position or "mixed base". Examples of degenerate bases are as described above.

According to certain embodiments of the invention, the oligonucleotide represented by formula (I) is a dual specificity oligonucleotide (referred to as DSO or DPO) as disclosed in WO 2006/095981. For details of the dual specificity oligonucleotide, refer to the above literature.

According to another specific embodiment of the invention, the oligonucleotide represented by formula (I) is a target discriminative (TD) probe as disclosed in WO 2011/028041. For details regarding the target discriminative probe, refer to the literature above.

The oligonucleotide of formula (I) provided in this step may be a pre-existing oligonucleotide (primer or probe).

Alternatively, the oligonucleotide of formula (I) provided in this step may be an oligonucleotide designed based on a target nucleic acid sequence to be amplified or detected.

The oligonucleotide of formula (I) is designed such that its X and Y portions have a sequence that can be substantially hybridized to the target nucleic acid sequence. To this end, the X and Y portions in the oligonucleotide of formula (I) are designed to match (to have significant sequence similarity) a particular region of the target nucleic acid sequence.

According to an embodiment of the present invention, the oligonucleotide is a Universal Base Primer (UBP). Specifically, the UBP has 1-3 universal base nucleotides; one or two of the universal base nucleotides are located in a core region in a range of third to sixth nucleotides at the 3'-end of the UBP, the rest is located in a range of fourth at the 5'-end of the UBP to seventh nucleotides at the 3'-end of the UBP, the universal base nucleotides being discontinuous at the UBP.

As used herein, "universal base primer" refers to a primer in which at least one nucleotide in the primer contains a universal base instead of a naturally occurring base (A, C, G, or T(U)). The UBP acts as an inhibitor for primer dimer formation. In particular, a primer including deoxyinosine or inosine among universal bases is referred to as an Inosine Primer (IPm).

As used herein, the term "core region" refers to an optimal range of positions for positioning one or two universal base nucleotides within a UBP to achieve inhibition of primer dimer formation, in particular, two-strand extendable primer dimer formation. That is, the core region refers to a specific region in the UBP in which one or two universal base nucleotides are located to exert the maximum effect.

For details regarding the universal base primer, refer to the information disclosed in WO 2006/095981.

The oligonucleotide used in the present invention may be an oligonucleotide designed to amplify and detect a plurality of nucleic acid sequences of a specific target nucleic acid molecule of a specific organism, or designed to have verified performance.

The oligonucleotide may be designed by hand or by a design program widely known in the art. Examples of the conventional primer/probe design program may include Primer3 (http://frodo.wi.mit.edu/), Visual OMP™ software (DNA Software, Inc., Ann Arbor, Mich.), Integrated DNA Technology (IDT) OligoAnalyzer 3.0 program (http://scitools.idtdna. com/Analvzer/oligocalc.asp), DINAmelt™ program (http://dinamelt.bioinfo.rpi.edu/), OLIGO 7 (Wojciech Rychlik (2007) "OLIGO 7 Primer Analysis Software" Methods Mol Biol. 402: 35-60), Primer Express 3.0 software (Applied Biosystems U.S.A), and the like, but are not limited thereto.

The oligonucleotide comprises a probe designed to satisfy at least one of the following conditions: (i) a Tm value of 50-85°C, (ii) a length of 15-50 nucleotides, (iii) exclusion of a mononucleotide $(G)_n$ run sequence; said n is at least 3, (iv) the 5'-end is G or C, (v) the GC content of the 5'-end portion is at least 40%.

Among the design conditions, the Tm value is, for example, 50-80°C, 50-75°C, 55-80°C, 55-75°C, 60-80°C, 60-75°C, 65-80°C, or 60-75°C. Specifically, the Tm value among the design conditions is 55-80°C, 60-78°C, 63-78°C, 65-75°C, 67-75°C, or 65-73°C.

The length of the design conditions is, for example, 10-60 nucleotides, 10-50 nucleotides, 10-45 nucleotides, 10-40 nucleotides, 10-35 nucleotides, 15-60 nucleotides, 15-50 nucleotides, 15-45 nucleotides, 15-40 nucleotides, 15-35 nucleotides, 20-60 nucleotides, 20-50 nucleotides, 20-45 nucleotides, 20-40 nucleotides, or 20-35 nucleotides.

Among the design conditions, for example, the mononucleotide $(G)_n$ run sequence having a n of at least 3 or 4 is excluded.

The GC content of the 5'-end portion of the probe is at least 40%, specifically 40-70% or 40-60%. The 5'-end portion refers to a region within 10 nucleotides from the 5'-end of the probe.

The oligonucleotide comprises a tagging probe designed to satisfy at least one of the following conditions: (i) the Tm value of the targeting portion 50-85°C, (ii) the length of the targeting portion 15-50 nucleotides, (iii) exclusion of at least three G-run sequences of the targeting portion, (iv) 5'-end of the targeting portion is G or C, (v) the GC content of the targeting portion 5'-end part includes at least 40%, (vi) the tagging portion length of the tagging portion 6-30 nucleotides, (vii) the mismatch sequence for the tagging portion length includes at least 30%, and (viii) the mismatch sequence for the tagging portion 3'-end part length includes at least 40%.

Among the design conditions for the targeting portion of the tagging probe, the Tm value, length, G-run sequence exclusion, G or C of 5'-end, and GC content of the 5'-end part may be described with reference to the description of the general probe.

The length of the tagging portion is specifically 6-20 nucleotides, 10-30 nucleotides, 10-20 nucleotides, 12-30 nucleotides, or 12-20 nucleotides.

The tagging portion should be sufficiently non-complementary to a specific region of the nucleic acid sequence in which the tagging probe is hybridized and should not be hybridized to the specific region under conditions in which the targeting portion of the tagging probe is hybridized. Specifically, the tagging portion includes a mismatch sequence of 40% or more, more specifically 50% or more, with respect to its length. Specifically, the 3'-end part of the tagging portion comprises at least 50% of the mismatching sequence with respect to its length.

The oligonucleotide comprises primers designed to satisfy at least one of the following conditions: (i) a Tm value of 40-70°C, (ii) a length of 15-60 nucleotides, and (iii) exclusion of the mononucleotide $(G)_n$ run sequence; wherein n is at least 3.

Among the design conditions, the Tm value is, for example, 40-70°C, 50-70°C, 55-70°C, 45-65°C, 50-65°C, 55-65°C, 45-60°C, or 50-65°C. Specifically, the Tm value among the design conditions is 40-70°C, 45-65°C, 50-65°C, 50-60°C, 55-65°C, or 55-60°C.

The length among the above design conditions is, for example, 15-60 nucleotides, 15-50 nucleotides, 15-45 nucleotides, 15-40 nucleotides, 15-35 nucleotides, 15-30 nucleotides, 15-25 nucleotides, 18-45 nucleotides, 18-40 nucleotides, 18-35 nucleotides, 18-30 nucleotides, or 18-25 nucleotides.

A criterion for a mononucleotide $(G)_n$ run sequence in the design conditions is, for example, exclusion of a mononucleotide $(G)_n$ run sequence having a n of at least 3 or 4.

If the primer is a DPO primer developed by the applicant (see U.S. Pat. No. 8092997), a description of the Tm and length of the DPO primer disclosed in the patent document can be presented as the design condition.

In order to perform an operation for providing alignment information, in the present step, the target nucleic acid sequence is provided as a m × 1 matrix comprising m nucleotides as matrix elements, the oligonucleotide sequence is provided as a 1 × n matrix comprising n nucleotides as matrix elements.

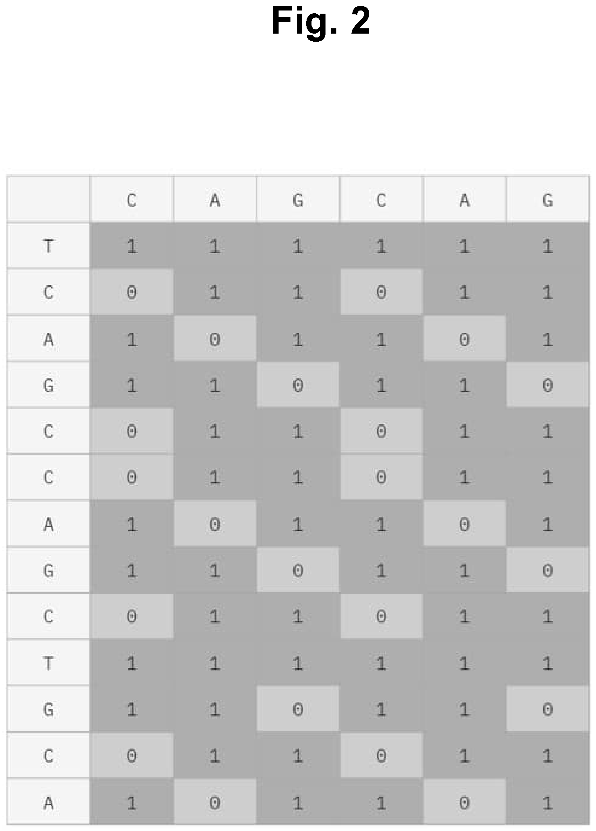

FIG. 2 shows a process of obtaining a match value or a mismatch value between nucleotides of a target nucleic acid sequence and an oligonucleotide sequence by performing a matrix operation of an oligonucleotide sequence on the target nucleic acid sequence in an operation table.

In FIG. 2, the target nucleic acid sequence (5'-TCAGCCAGCTGCA-3'; SEQ ID NO: 1) provided in the 13×1 matrix can be identified in the leftmost column, and the oligonucleotide sequence (5'-CAGCAG-3'; SEQ ID NO: 2) provided in the 1×6 matrix can be identified in the topmost row.

The nucleotides included in the target nucleic acid sequence and the oligonucleotide are expressed in 4 bits for each base type.

According to the present invention, in order to determine a match or mismatch between nucleotides of a target nucleic acid sequence and an oligonucleotide sequence, nucleotides are expressed in 4 bits for each type of base, and a match or mismatch between nucleotides is confirmed through a bit operation, rather than confirming a string match for confirming whether characters between bases are the same.

According to an embodiment of the present invention, the base includes a degenerate base and/or a universal base. Descriptions of degenerate bases and universal bases are as described above.

In the present specification, the term "4 bits" means a number representing each number of 4 digits as a binary number.

According to IUPAC nucleotide codes, natural bases include adenine (A), guanine (G), cytosine (C), and thymine (T) (uracil (U)), and degenerate bases include R (A or G), Y (C or T), S (G or C), W (A or T), K (G or T), M (A or C), B (C, G or T), D (A, G or T), H (A, C or T), V (A, C or G), and N (A, C, G or T, that is, any base). Meanwhile, examples of the universal base may include Inosine (I).

Nucleotides may be expressed randomly in 4 bits for each type of base, but may be expressed with a certain rule. For example, if the natural base is represented by 4 bits in the order of adenine (A), guanine (G), cytosine (C), and thymine (T) (uracil (U)), which are natural bases, adenine (A) may be represented by 1000, guanine (G) may be represented by 0100, cytosine (C) may be represented by 0010, and thymine (T) and uracil (U) may be represented by 0001, and considering this rule, degenerate bases may be represented by 4 bits: R(A or G): 1100; Y(C or T): 0011; S(G or C): 0110; W(A or T): 1001; K(G or T): 0101; M(A or C): 1010; B(C, G or T): 0111; D(A, G or T): 1101; H(A, C or T): 1011; V(A, C or G): 1110; N(A, C, G or T(any base)): 1111. In addition, Inosine (I), which is a universal base, may be expressed as 1111. On the other hand, a 4-bit representation of a base in the reverse direction may be written in the opposite direction of the 4-bit. For example, the reverse adenine (A) of the adenine (A) 1000 is expressed as 0001.

Step (b): performing a matrix operation to obtain a match value or mismatch value between nucleotides of the target nucleic acid sequence and the oligonucleotide sequence **(120)**

Subsequently, the method of the present invention comprises (b) performing a matrix operation of the oligonucleotide sequence for the target nucleic acid sequence to obtain a match value or mismatch value between nucleotides of the target nucleic acid sequence and the oligonucleotide sequence. The matrix operation is a multiplication of the $m \times 1$ matrix and the $1 \times n$ matrix, and an AND operation of 4 bits of the nucleotide that is an element of the $m \times 1$ matrix and 4 bits of the nucleotide that is an element of the $1 \times n$ matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score.

The matrix operation is the multiplication of the $m \times 1$ matrix and the $1 \times n$ matrix, resulting in the $m \times n$ matrix.

In the present specification, the bit AND operation indicates 1 when the numbers of each digit are the same as 1, and 0 when the numbers of each digit are different as 1 and 0 or are the same as 0. For example, bit AND operation values of 0010 and 0011 are 0010, and bit AND operation values of 1010 and 0100 are 0000.

According to an embodiment of the present invention, the predetermined match score and the mismatch score are integers. Specifically, the predetermined match score is an integer selected from -20 to 20, more specifically an integer selected from -10 to 10, and still more specifically an integer selected from -5 to 5.

In addition, the predetermined mismatch score is specifically an integer selected from -20 to 20, more specifically an integer selected from -10 to 10, and still more specifically an integer selected from -5 to 5.

In FIG. 2, a predetermined match score is set to 0, and a predetermined mismatch score is set to 1.

According to an embodiment, the predetermined match score and mismatch score are the same as or different from each other. More specifically, the predetermined match score and mismatch score are different from each other. For example, when the match score is set to 0, the mismatch score may be set to 1, and vice versa.

According to an embodiment of the present invention, when the matrix operation value is same as 4 bits of the nucleotide of the target nucleic acid sequence among the nucleotides on which the matrix operation is performed, the nucleotides on which the matrix operation is performed represent being a match, and the match value is a value expressing the match as a predetermined match score.

According to an embodiment of the present invention, when the matrix operation value is same as 4 bits of the nucleotide of the oligonucleotide sequence among the nucleotides on which the matrix operation is performed, the nucleotides on which the matrix operation is performed represent being a match, and the match value is a value expressing the match as a predetermined match score.

According to an embodiment, the method further comprises that when the matrix operation value is different from 4 bits of the nucleotide of the target nucleic acid sequence among the nucleotides on which the matrix operation is performed, the nucleotides on which the matrix operation is performed represent being a mismatch, and the mismatch value is a value expressing the mismatch as a predetermined mismatch score.

According to an embodiment of the present invention, the step (b) further includes that when the matrix operation value is same as 4 bits of the nucleotide of the target nucleic acid sequence among the nucleotides on which the matrix operation is performed, the nucleotides on which the matrix operation is performed represent being a match, and the match value is a value expressing the match as a predetermined match score, and when the matrix operation value is different from 4 bits of all of the nucleotides on which the matrix operation is performed or the matrix operation value is different from 4 bits of the nucleotide of the target nucleic acid sequence on which the matrix operation is performed, the nucleotides on which the matrix operation is performed represent being a mismatch, and the mismatch value is a value expressing the mismatch as a predetermined mismatch score.

FIG. 2 shows a process of obtaining a match value or a mismatch value between nucleotides of a target nucleic acid sequence and an oligonucleotide sequence by performing a matrix operation of an oligonucleotide sequence on the target nucleic acid sequence in an operation table, and the operation table includes components ($C_{i,j}$, $1 \leq i \leq m$, $1 \leq j \leq n$, m is 13, n is 6) of a m×n matrix by multiplying the m×1 matrix and a 1×n matrix. In FIG. 2, the predetermined match score is 0, and the predetermined mismatch score is 1.

In FIG. 2, the element or component ($C_{1,1}$) of the first row and the first column among the elements or components of the 13×6 matrix indicates a matrix operation value of 0000 by a matrix operation (multiplication of the matrix and bit AND operation) of base T (0001) of the component ($A_{1,1}$) of the 13×1 matrix and base C (0010) of the component ($B_{1,1}$) of the 1×6 matrix. The matrix operation value of 0000 is different from 4 bits 0001 of base T of the component ($A_{1,1}$) of the 13×1 matrix and 4 bits 0010 of base C of the component ($B_{1,1}$) of the 1×6 matrix, and thus the base of the element ($A_{1,1}$) of the 13×1 matrix and the base of the element ($B_{1,1}$) of the 1×6 matrix indicates mismatch, and at this time, 1 is given as the mismatch value.

In FIG. 2, the component ($C_{2,1}$) of the second row and the first column of the components of the 13×6 matrix indicates a matrix operation value of 0010 by a matrix operation (multiplication of the matrix and bit AND operation) of base C (0010) of the component ($A_{2,1}$) of the 13×1 matrix and base C (0010) of the component ($B_{1,1}$) of the 1×6 matrix and the matrix operation value of 0010 is the same as 4 bits 0010 of base C of the component ($A_{2,1}$) of the 13×1 matrix and 4 bits 0010 of base C of the component ($B_{1,1}$) of the 1×6 matrix. Thus, the base of the component ($A_{2,1}$) of the 13×1 matrix and the base of the component ($B_{1,1}$) of the 1×6 matrix indicates a match, and at this time, 0 is given as the match value.

In this way, a match value or a mismatch value of the operation table of FIG. 2 is obtained.

If the base of the component $B_{1,1}$ of the 1×6 matrix in FIG. 2 is the degenerate base W(A or T) 1001, a matrix operation value of 0001 is calculated by the matrix operation (multiplication of the matrix and bit AND operation) of the base T 0001 of the component $A_{1,1}$ of the 13×1 matrix and the base W(A or T) 1001 of the component $B_{1,1}$ of the 1×6 matrix, the matrix operation value of 0001 is the same as 4 bits 0001 of base T of the component ($A_{1,1}$) of the 13×1 matrix. Thus, the base of the component ($A_{1,1}$) of the 13×1 matrix and the base of the component ($B_{1,1}$) of the 1×6 matrix indicates a match, and at this time, 0 is given as the match value.

Step (c): <u>performing a trace operation of a square matrix to obtain the sum of match values and/or mismatch values of diagonal elements of the square matrix (130)</u>

And then the method of the present invention comprises (c) performing a trace operation of a n × n square matrix in the m × n matrix on which the matrix operation is performed to obtain the sum of match values and/or mismatch values of diagonal elements of the n × n square matrix.

In the present specification, the term "diagonal sum (trace) operation" refers to an operation of obtaining a sum of diagonal components of a n×n square matrix.

In this step, by performing a diagonal sum operation, it is possible to quickly provide semi-global alignment information without considering a gap in the aligned target nucleic acid sequence and oligonucleotide sequence.

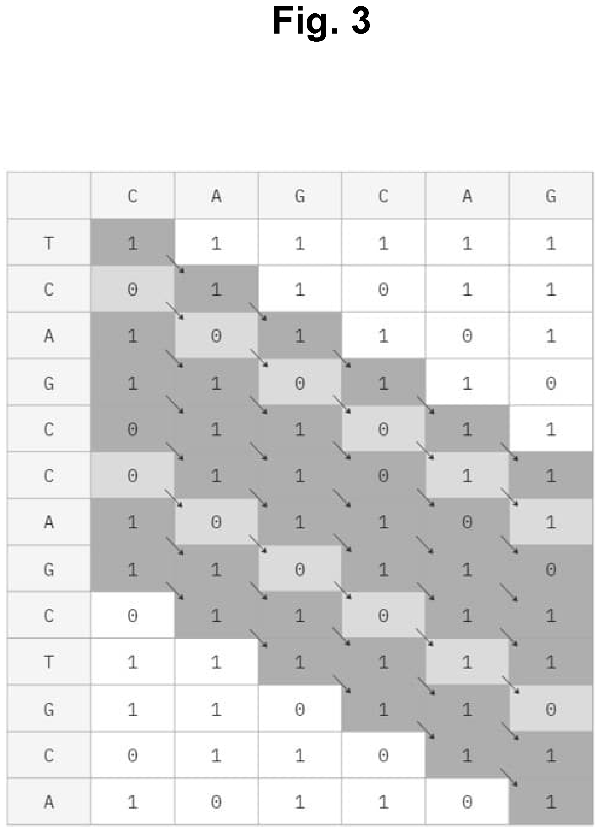

FIG. 3 illustrates a process of calculating a sum of match values and/or mismatch values of diagonal elements of a 6×6 square matrix by performing a diagonal sum (trace) operation of the 6×6 square matrix in the 13×6 matrix in which the matrix operation is performed.

In Fig. 3, an n × n square matrix (n is 6) can be formed from a target nucleic acid sequence that is an m × 1 matrix (m is 13) and an oligonucleotide sequence that is a 1 × n matrix (n is 6). Here, a plurality of target nucleic acid sequences that are n × 1 matrices (n is 6) can be formed. Accordingly, it can be seen in FIG. 3 that the diagonal sum (trace) operation of the 6×6 square matrix can be performed eight times in the 13×6 matrix in which the matrix operation is performed.

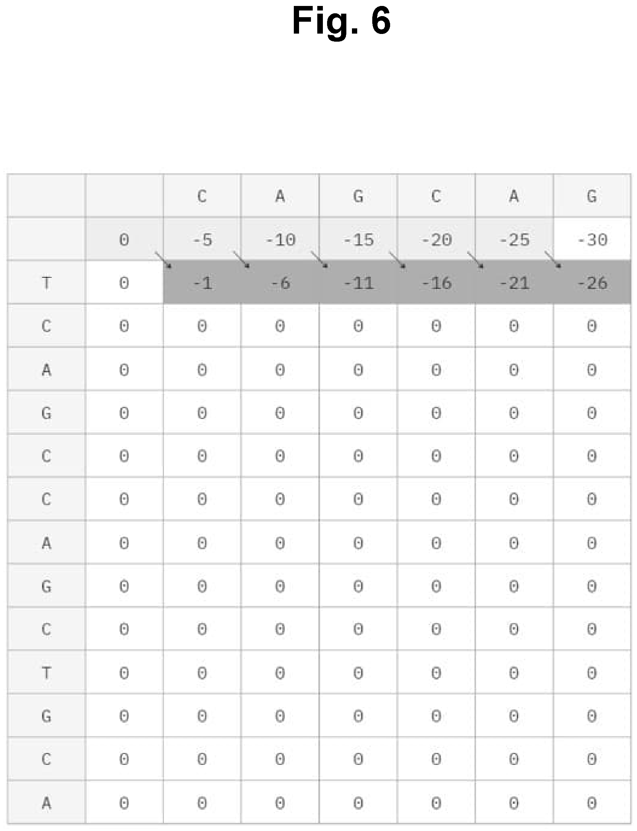

As a result of performing the diagonal sum operation of the first 6×6 square matrix in FIG. 3, 6 is calculated by summing all of the mismatch value (1) of the component $C_{1,1}$ of the matrix, the mismatch value (1) of $C_{2,2}$, the mismatch value (1) of $C_{3,3}$, the mismatch value (1) of $C_{4,4}$, the mismatch value (1) of $C_{5,5}$, and the mismatch value (1) of $C_{6,6}$ of the matrix, and as the diagonal sum operation result of the second 6×6 square matrix in FIG. 3, 2 is calculated by summing all of the match value (0) of the component $C_{2,1}$ of the matrix, the match value (0) of $C_{3,2}$, the match value (0) of $C_{4,3}$, the match value (0) of $C_{5,4}$, the mismatch value (1) of $C_{6,5}$, and the mismatch value (1) of $C_{7,6}$. As described above, the results of performing the diagonal sum operation of the third, fourth, and fifth 6×6 square matrices are calculated as 3, 6, and 5, respectively.

As a result of performing the diagonal sum operation of the sixth 6×6 square matrix in FIG. 3, 1 is calculated by summing all of the match value (0) of the component $C_{6,1}$ of the matrix, the match value (0) of $C_{7,2}$, the match value (0) of $C_{8,3}$, the match value (0) of $C_{9,4}$, the mismatch value (1) of $C_{10,5}$, and the match value (0) of $C_{11,6}$, and as a result of performing the diagonal sum operation of the seventh 6×6 square matrix in this way, 6 is calculated.

Finally, as a result of performing the diagonal sum operation of the eighth 6×6 square matrix in FIG. 3, 6 is calculated by summing all of the mismatch value 1 of the component $C_{8,1}$ of the matrix, the mismatch value 1 of $C_{9,2}$, the mismatch value 1 of $C_{10,3}$, the mismatch value 1 of $C_{11,4}$, the mismatch value 1 of $C_{12,5}$, and the mismatch value 1 of $C_{13,6}$.

Step (d): <u>providing alignment information of the nucleotides of the target nucleic acid sequence of row elements and the nucleotides of the oligonucleotide sequence of column elements corresponding to the diagonal elements of the square matrix having the sum satisfying the predetermined threshold value</u> **(140)**

Finally, the method of the present invention comprises (d) when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold value. The alignment information comprises information selected from the group consisting of a match and/or mismatch between nucleotides of the target nucleic acid sequence and the oligonucleotide sequence, an alignment pattern comprising the match and/or mismatch information, the number of the mismatch, and the position of the oligonucleotide sequence in aligned target nucleic acid sequences.

The predetermined match or mismatch threshold value may be expressed as the number of matches or mismatches, or as a ratio of the number of matches or mismatches to the nucleotide length of the oligonucleotide sequence. When the predetermined match or mismatch threshold value is represented by the number of matches or mismatches, it may be determined as an absolute threshold, and when the predetermined match or mismatch threshold value is represented as a ratio of the number of matches or mismatches to the nucleotide length of the oligonucleotide sequence, it may be determined as a relative threshold.

The predetermined match threshold value may be selected from 3 to 60 when expressed as the number of matches. The predetermined match threshold value may be selected from 5% to 100% when expressed as a ratio of the number of matches to the nucleotide length of the oligonucleotide sequence.

The predetermined mismatch threshold value may be selected from 0 to 40 when expressed by the number of mismatches. The predetermined mismatch threshold value may be selected from 0% to 60% when expressed as a ratio of the number of mismatches to the nucleotide length of the oligonucleotide sequence.

Alternatively, the predetermined match or mismatch threshold value may be determined according to the match or mismatch value. For example, when the number of matches or mismatch values is greater than 0, the match or mismatch threshold value may be selected from the number of 1 to 100. When the match or mismatch value is 0, the match or mismatch threshold value is 0, and when the match or mismatch value is a number less than 0, the match or mismatch threshold value may be selected from the number of -1 to -100.

According to an embodiment of the present invention, that the sum satisfies a predetermined match threshold value represents that the sum is greater than or equal to a predetermined match threshold value, and that the sum satisfies a predetermined mismatch threshold value represents that the sum is less than or equal to a predetermined mismatch threshold value. For example, when the predetermined match threshold value is 2, the sum is at least 2, and when the predetermined mismatch threshold value is 2, the sum is at most 2.

In FIG. 3, when the mismatch value is 1 and the match value is 0, the diagonal sum operation value of the first 6×6 square matrix is 6, the diagonal sum operation value of the second 6×6 square matrix is 2, the diagonal sum operation value of the third 6×6 square matrix is 3, the diagonal sum operation value of the fourth 6×6 square matrix is 6, the diagonal sum operation value of the fifth 6×6 square matrix is 5, the diagonal sum operation value of the sixth 6×6 square matrix is 1, and the diagonal sum operation value of the last eighth 6×6 square matrix is 6.

In this case, if the predetermined mismatch threshold value is 2, the diagonal sum operation values of the second and sixth square matrices having a value of 2 or less satisfy the predetermined mismatch threshold value, and alignment information of the nucleotide of the target nucleic acid sequence of the row component and the nucleotide of the oligonucleotide sequence of the column component corresponding to the diagonal components of the second and sixth 6×6 square matrices having the sum satisfying the predetermined threshold value is provided as shown in Table 1 below:

Table 1

| Patterns | Match type | SEQ ID NO |
|---|---|---|
| CAGCAG<br>\| \| \| \| XX<br>TCAGCCAGCTGCA | 2\|N | SEQ ID NO:2<br><br>SEQ ID NO:1 |
| CAGCAG<br>\| \| \| \| X\|<br>TCAGCCAGCTGCA | 1\|N | SEQ ID NO:2<br><br>SEQ ID NO:1 |

In Table 1, the vertical bar (|) of the pattern indicates a match between aligned nucleotides, X indicates a mismatch between aligned nucleotides, and in the match type, the vertical bar N (|N) indicates a typical oligonucleotide rather than a DPO primer having a separation portion, and the number in front of the vertical bar (|) indicates the number of mismatches. If 0 or number is written after the vertical bar (|) in the match type, the match type indicates the match type of the DPO primer.

In the present specification, "providing alignment information" includes displaying alignment information on an output device (*e.g.,* a user terminal), allowing users to download the alignment information to a storage medium, or establishing or building the alignment information into a database in a computer.

In this way, it is possible to provide semi-global alignment information of the oligonucleotide to the target nucleic acid sequence without considering the gap.

The sequence alignment algorithm or program may be largely divided into a local alignment, a global alignment, and a semi-global alignment.

The local alignment locally checks whether there is a mismatch between the query sequence and the sequence of the subject, and provides match or mismatch information even when a match or mismatch result satisfying a cut-off value input as a parameter is in a part of the query sequence. As a result, the entire match or mismatch information of the query sequence may not be known, and a plurality of match or mismatch results of the query sequence with respect to the sequence of the subject are shown.

In order to provide coverage for the entire oligonucleotide sequence (the entire length), a repair method is additionally required to overcome the problem caused by the characteristic of the local alignment (specifically, checking whether there is a local mismatch), but when the number of nucleic acid sequences in the related art is very large, there is a problem in that it is not possible to show a match or mismatch pattern for the entire oligonucleotide due to a failure of a memory or the like.

The global alignment checks whether there is a mismatch between the query sequence and the sequence of the subject, and provides one match or mismatch information when there is a match or mismatch result satisfying the cut-off value input as a parameter in the entire sequence of the query sequence and the sequence of the subject. Therefore, match or mismatch information of the entire query sequence is provided through the match or mismatch result of some of the query sequences for the entire sequence of the subject.

Semi-global alignment checks whether there is a mismatch between the entire query sequence and partial sequence of the subject, and shows a match or mismatch result when there is a match or mismatch result that satisfies the cut-off value input as a parameter. Specifically, the semi-global alignment is a sequence alignment algorithm or program performed by global alignment for query sequences and local alignment for subjects.

In the present invention, a program including a repair method is not required to overcome the problem of checking whether a local alignment (specifically, BLAST) is locally mismatched through an algorithm for implementing a semi-global alignment. That is, according to the present invention, since match or mismatch information can be provided for the entire oligonucleotide without using the repair method for a target nucleic acid sequence by using semi-global alignment, fewer computer resources can be used than when using local alignment in the related art, and an improved effect is also shown in the rate of providing alignment information.

By using semi-global alignment, it is possible to check whether there is a mismatch between regions of the target nucleic acid sequence corresponding to the entire length of the oligonucleotide. For example, in the case of a tagging probe including a tagging portion and a targeting portion, it is not possible to check whether there is a mismatch between the targeting portion of the tagging probe and a region of a nucleic acid sequence corresponding thereto and whether the tagging portion is mismatched, by using conventional local alignment, but according to the present invention, it is possible to check whether there is a mismatch with respect to the entire tagging probe.

Further, in the case of the DPO primer including the 5'-high Tm specific portion (X)-separation portion (Y)-3'-low Tm specific portion (Z), an algorithm or program using a conventional local alignment and repair method may check whether there is a mismatch between the 5'-high Tm specific portion (X) and a region of a corresponding nucleic acid sequence using local alignment, do not check whether there is a mismatch between the separation portion (Y) and a region of a corresponding nucleic acid sequence, and check whether there is a mismatch between the 3'-low Tm specific portion (Z) and a region of a corresponding nucleic acid sequence using the repair method. However, according to the present invention using semi-global alignment, alignment information on the entire length of the DPO primer may be provided more quickly.

According to an embodiment of the present invention, the method provides coverage of oligonucleotides for a plurality of target nucleic acid sequences.

By performing the method of the present invention on each of a plurality of target nucleic acid sequences, it is possible to provide the coverage of oligonucleotides for the plurality of target nucleic acid sequences.

The plurality of target nucleic acid sequences are at least 3, at least 5, at least 10, at least 20, at least 30, at least 40, at least 50, at least 60, at least 70, at least 80, at least 90, or at least 100 target nucleic acid sequences.

The plurality of target nucleic acid sequences in the present invention are target nucleic acid sequences having sequence similarity. Specifically, the target nucleic acid sequences having the sequence similarity may be a plurality of target nucleic acid sequences of one target nucleic acid molecule or a plurality of target nucleic acid sequences of two or more target nucleic acid molecules.

According to an embodiment, the plurality of target nucleic acid sequences in the present invention are a plurality of nucleic acid sequences having sequence similarity of one target nucleic acid molecule having genetic diversity.

For example, the plurality of target nucleic acid sequences used in the present invention are a plurality of nucleic acid sequences having sequence similarity of a target nucleic acid molecule exhibiting genetic diversity, such as a genome sequence of a virus. For example, if an influenza A virus is to be detected and the M gene is designated as a target nucleic acid molecule, the diversity target nucleic acid sequences of the M gene of the influenza A virus may be used in the present invention.

More specifically, the plurality of target nucleic acid sequences are a whole genome sequence of a virus or bacteria having genetic diversity, a partial sequence of a genome, or a plurality of nucleic acid sequences of one gene.

The plurality of target nucleic acid sequences may be included in a nucleotide database and provided. The nucleotide database used in the present invention refers to a set of data related to two or more nucleotide sequences derived from various sources, and may be used interchangeably with the terms "database of nucleotide sequences", "nucleotide sequence database", or "database".

The nucleotide database may include information related to a nucleotide sequence (*e.g.,* a specific sequence thereof, a taxanomy ID, a taxonomy name, an organism name, an access number, and the like).

The database may be available to the public, commercially available, or generated by the inventor. The database is a set arranged for the convenience and speed of computer search.

Examples of databases known in the art include, but are not limited to, GenBank databases, EST databases, EMBL nucleotide sequence databases, Entrez nucleotide databases, LIFESEQ™ databases, and GISAID databases.

As used herein, the term "coverage" refers to providing information on target nucleic acid sequences that are hybridized or covered with an oligonucleotide or have a sequence similar to the oligonucleotide sequence. The information on the target nucleic acid sequences comprises information on the number of target nucleic acid sequences and the access number of the target nucleic acid sequences (Accession No.), a taxanomy name to which a target nucleic acid sequence belongs, a taxonomy ID assigned to the taxonomy name, a ratio of target nucleic acid sequences hybridized or covered with oligonucleotides to the entire target nucleic acid sequence, the number of matches or mismatches of the oligonucleotides, or a mismatch pattern of the oligonucleotides.

## Recording medium, device, and program

In another aspect of the present invention, there is provided a computer readable storage medium containing instructions to configure a processor to perform a method for providing alignment information of an oligonucleotide for a target nucleic acid sequence, the method comprising:

(a) providing a target nucleic acid sequence comprising m nucleotides and an oligonucleotide sequence comprising n nucleotides; wherein the target nucleic acid sequence is provided as a $m \times 1$ matrix comprising m nucleotides as matrix elements, the oligonucleotide sequence is provided as a $1 \times n$ matrix comprising n nucleotides as matrix elements, and the nucleotides are expressed in 4 bits for each base type,

(b) performing a matrix operation of the oligonucleotide sequence for the target nucleic acid sequence to obtain a match value or mismatch value between nucleotides of the target nucleic acid sequence and the oligonucleotide sequence; wherein the matrix operation is a multiplication of the $m \times 1$ matrix and the $1 \times n$ matrix, and an AND operation of 4 bits of the nucleotide that is an element of the $m \times 1$ matrix and 4 bits of the nucleotide that is an element of the $1 \times n$ matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score,

(c) performing a trace operation of a n × n square matrix in the m × n matrix on which the matrix operation is performed to obtain the sum of match values and/or mismatch values of diagonal elements of the n × n square matrix; and

(d) when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold value; wherein the alignment information comprises information selected from the group consisting of a match and/or mismatch between nucleotides of the target nucleic acid sequence and the oligonucleotide sequence, an alignment pattern comprising the match and/or mismatch information, the number of the mismatch, and the position of the oligonucleotide sequence in aligned target nucleic acid sequences.

In still another aspect of the present invention, there is provided a computer program to be stored on a computer readable storage medium, to configure a processor to perform a method for providing alignment information of an oligonucleotide for a target nucleic acid sequence, the method comprising:

(a) providing a target nucleic acid sequence comprising m nucleotides and an oligonucleotide sequence comprising n nucleotides; wherein the target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type,

(b) performing a matrix operation of the oligonucleotide sequence for the target nucleic acid sequence to obtain a match value or mismatch value between nucleotides of the target nucleic acid sequence and the oligonucleotide sequence; wherein the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score,

(c) performing a trace operation of a n × n square matrix in the m × n matrix on which the matrix operation is performed to obtain the sum of match values and/or mismatch values of diagonal elements of the n × n square matrix; and

(d) when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold value; wherein the alignment information comprises information selected from the group consisting of a match and/or mismatch between nucleotides of the target nucleic acid sequence and the oligonucleotide sequence, an alignment pattern comprising the match and/or mismatch information, the number of the mismatch, and the position of the oligonucleotide sequence in aligned target nucleic acid sequences.

In another aspect of the present invention, there is provided a device for providing alignment information of an oligonucleotide relative to a target nucleic acid sequence, comprising (a) a computer processor and (b) a computer readable storage medium of the present invention coupled to the computer processor.

Since the storage medium, the device, and the computer program of the prevent invention are intended to perform the present methods described as above in a computer, the common descriptions among them are omitted in order to avoid undue redundancy leading to the complexity of this specification.

The program instructions are operative, when performed by the processor, to cause the processor to perform the present method described above. The program instructions for performing a method for providing alignment information of an oligonucleotide for a target nucleic acid sequence may comprise the following instructions: (i) an instruction to provide a target nucleic acid sequence comprising m nucleotides and an oligonucleotide sequence comprising n nucleotides; (ii) an instruction to perform a matrix operation of the oligonucleotide sequence for the target nucleic acid sequence to obtain a match value or mismatch value between nucleotides of the target nucleic acid sequence and the oligonucleotide sequence; (iii) an instruction to perform a trace operation of a n × n square matrix in the m × n matrix on which the matrix operation is performed to obtain the sum of match values and/or mismatch values of diagonal elements of the n × n square matrix; and (iv) when the sum satisfies a predetermined match or mismatch threshold value, an instruction to provide (*e.g.,* to display on an output device) alignment information of the nucleotides of the target nucleic acid sequence of row elements and the nucleotides of the oligonucleotide sequence of column elements corresponding to the diagonal elements of the n × n square matrix having the sum satisfying the predetermined threshold value.

The method of the present invention is implemented in a processor, wherein the processor may be a processor in a stand-alone computer, a network attached computer, or a data acquisition device, such as a real-time PCR device.

The types of the computer readable storage medium include various storage media, for example, CD-R, CD-ROM, DVD, flash memory, floppy disk, hard drive, portable HDD, USB, magnetic tape, MINIDISC, nonvolatile memory card, EEPROM, optical disk, optical storage medium, RAM, ROM, system memory and web server, but are not limited thereto.

According to an embodiment of the present invention, the computer readable storage medium is a non-transitory computer readable storage medium.

Alignment information of the oligonucleotide with respect to the target nucleic acid sequence may be provided in various ways. For example, the alignment information of the oligonucleotide for the target nucleic acid sequence may be provided to a separate system, such as a desktop computer system, via a network connection (*e.g.,* LAN, VPN, intranet, and internet) or a direct connection (*e.g.,* USB or other direct wired or wireless connection), or may be provided on a portable medium such as a CD, DVD, floppy disk and portable HDD. Similarly, the oligonucleotides may be provided to a server system via a network connection (*e.g.,* LAN, VPN, internet, intranet and wireless communication network) to a client, such as a notebook or a desktop computer system.

The instructions to configure the processor to perform the present invention may be included in a logic system. The instructions may be downloaded and stored in a memory module (*e.g.,* hard drive or other memory, such as a local or attached RAM or ROM), although the instructions can be provided on any software storage medium (*e.g.,* portable HDD, USB, floppy disk, CD and DVD). A computer code for implementing the present invention may be implemented in a variety of coding languages, such as C, C++, Java, Visual Basic, VBScript, JavaScript, Perl and XML. In addition, a variety of languages and protocols may be used in external and internal storage and transmission of data and commands according to the present invention.

The computer processor may be prepared in such a manner that a single processor can do several performances. Alternatively, the processor unit may be prepared in such a manner that several processors do the several performances, respectively.

## II. Method for Providing Alignment Information in Consideration of a Gap (Second Aspect)

In another aspect of the present invention, there is provided a computer-implemented method for providing alignment information of an oligonucleotide for a target nucleic acid sequence comprising:

(a) providing a target nucleic acid sequence comprising m nucleotides and an oligonucleotide sequence comprising n nucleotides; wherein the target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type,

(b) providing an operation table; wherein the operation table comprises a part where operation is performed and a part where a gap score is input, the part where operation is performed comprises elements ($C_{i,j}$, $1{\le}i{\le}m$, $1{\le}j{\le}n$) of the m×n matrix obtained by multiplying the m×1 matrix and the 1×n matrix, and the part where a gap score is input comprises an origin point ($C_{0,0}$), 0 row elements ($C_{0,j}$, $1{\le}j{\le}n$), and 0 column elements ($C_{i,0}$, $1{\le}i{\le}m$),

(c) inputting a gap score into the part where a gap score is input of the operation table; wherein origin point value + (j x gap score (k)) ($1{\le}j{\le}n$, k is an integer) is input for each column element in the 0 row elements of the part where a gap score is input, and origin point value + (i x gap score (k)) ($1{\le}i{\le}n$, k is an integer) is input for each row element in the 0 column elements of the part where a gap score is input,

(d) performing an operation and inputting an operation value into the part where an operation is performed of the operation table; wherein the operation is performed by a method comprising the following steps for each row in order from the first row to the last row of the part where an operation is performed,

d-1) obtaining a maximum value of any one operation value of addition selected from i-1) an operation value of addition in the relationship with an above matrix element and i-2) an operation value of addition in the relationship with a left matrix element, with i-3) an operation value of addition in the relationship with a left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-2) the operation value of addition in the relationship with the left matrix element is an operation value of addition obtained by adding the gap score (k, k is an integer) to the value of the left matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with any one operation value of addition from i-1) and i-2) not selected in step d-1), respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element;

(e) performing backtracking according to the indication in step d-2) from the matrix element that satisfies a predetermined score threshold value among the operation values of the matrix elements in the last column of the part where an operation is performed where the operation value is input; and

(f) providing alignment information between nucleotides of the target nucleic acid sequence and nucleotides of the oligonucleotide sequence according to the origin of the operation value of the backtracked matrix element; wherein the alignment information comprises information selected from the group consisting of a match, a mismatch, and/or a gap between nucleotides of the target nucleic acid sequence and the oligonucleotide sequence, an alignment pattern comprising the match, mismatch, and/or gap information, the number of the mismatch, and the position of the oligonucleotide sequence in aligned target nucleic acid sequence.

The present inventors have attempted to develop a method for efficiently using computer resources and speeding up the provision of alignment information in providing alignment information of oligonucleotides for a target nucleic acid sequence. As a result, according to the present invention, that alignment information with respect to the entire length of the oligonucleotides can be provided more accurately and rapidly with less computer resources than the conventional method is confirmed by developing a technique capable of providing semi-global alignment of oligonucleotides with respect to a target nucleic acid sequence by applying dynamic programming.

The method of a second aspect of the present invention is directed to a technique for providing alignment information of an oligonucleotide relative to a target nucleic acid sequence in consideration of gaps.

When the target nucleic acid sequence and the oligonucleotide sequence are aligned, a gap (or gaps) is formed in the target nucleic acid sequence and/or the oligonucleotide sequence in order to show an alignment result showing a predetermined similarity. Accordingly, the method of the second aspect of the present invention is intended to provide alignment information in consideration of the gap.

As used herein, "providing alignment information in consideration of a gap" means providing alignment information in which a gap may be included.

"Dynamic programming" used in the present specification is a technique of optimization theory, and is an algorithm design technique for efficiently obtaining a value by using a value in a different range to obtain a value up to a specific range. In other words, dynamic programming collectively refers to an algorithm that considers a problem as an extension of a smaller problem and uses a solution obtained in the past to solve the problem.

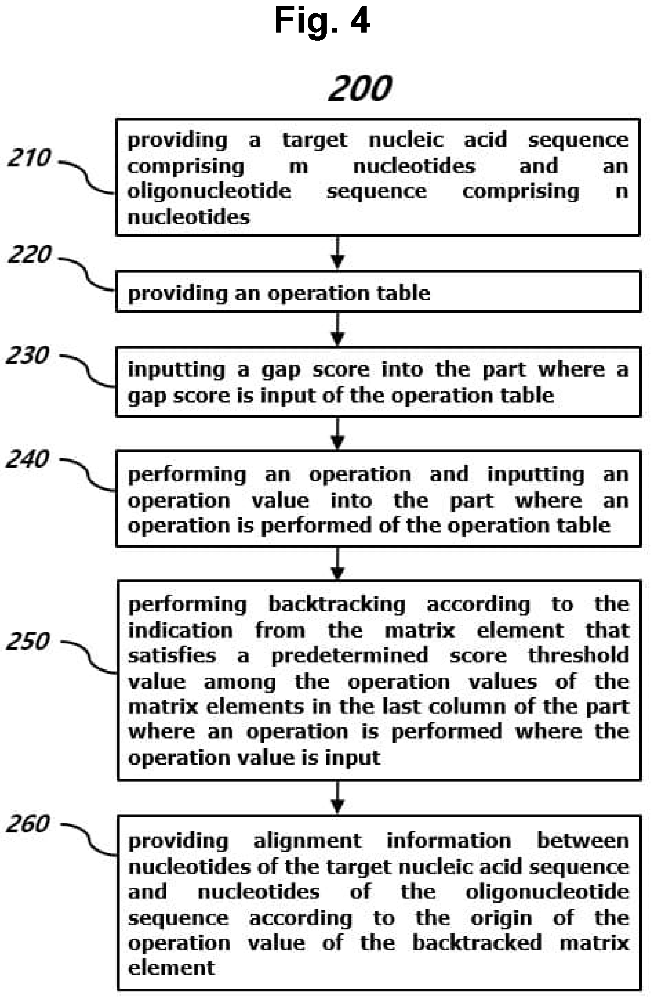

FIG. 4 is a flowchart of processes of performing a method of the present invention according to another embodiment of the present invention. The method of the present invention will be described with reference to FIG. 4 as follows: Step (a): providing a target nucleic acid sequence and an oligonucleotide sequence **(210)**

First, the method of the present invention comprises (a) providing a target nucleic acid sequence comprising m nucleotides and an oligonucleotide sequence comprising n nucleotides. The target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type.

In the method of the present invention of the second aspect, step (a) may be described with reference to the description of step (a) of the first aspect described above. Therefore, the description of the common contents among them will be omitted in order to avoid excessive complexity of the present specification.

Although it is described herein that m nucleotides of a target nucleic acid sequence are provided as a m×1 matrix and n nucleotides of an oligonucleotide sequence are provided as a $1 \times n$ matrix, it is clearly understood by those skilled in the art that m nucleotides of a target nucleic acid sequence are provided as a $1 \times m$ matrix and n nucleotides of an oligonucleotide sequence are provided as a $n \times 1$ matrix, thereby performing the method of the present invention described below. It is also clearly understood by those skilled in the art that methods of the present invention performed by providing m nucleotides of a target nucleic acid sequence as a $1 \times m$ matrix and n nucleotides of an oligonucleotide sequence as a $n \times 1$ matrix are also included in the scope of the present invention.

That is, it may be clearly understood by those skilled in the art that the method of the present invention performed by providing m nucleotides of a target nucleic acid sequence as a $m \times 1$ matrix, and providing n nucleotides of an oligonucleotide sequence as a $1 \times n$ matrix and the method of the present invention performed by providing m nucleotides of a target nucleic acid sequence as a $1 \times m$ matrix, and providing n nucleotides of an oligonucleotide sequence as a $n \times 1$ matrix, are considered equivalent under the doctrine of equivalents.

When the order of the rows and columns of the target nucleic acid sequence and the oligonucleotide sequence is changed, the order is changed and performed in a process to be described later.

According to an embodiment of the present invention, the m is a natural number.

According to an embodiment, the oligonucleotide is a primer or a probe.

According to an embodiment of the present invention, the n is a natural number.

According to an embodiment, the m has a number greater than n.

According to an embodiment of the present invention, the oligonucleotide is an oligonucleotide represented by the following formula (I):

$$5\text{'-X-Y-Z-3'} \qquad (I)$$

wherein, X represents a portion comprising a hybridizing nucleotide sequence that is hybridized with the target nucleic acid sequence, Y represents a separation portion comprising two or more consecutive bases that do not involve in Watson-Crick base pairing, and Z represents a portion comprising a hybridizing nucleotide sequence that is hybridized with the target nucleic acid sequence.

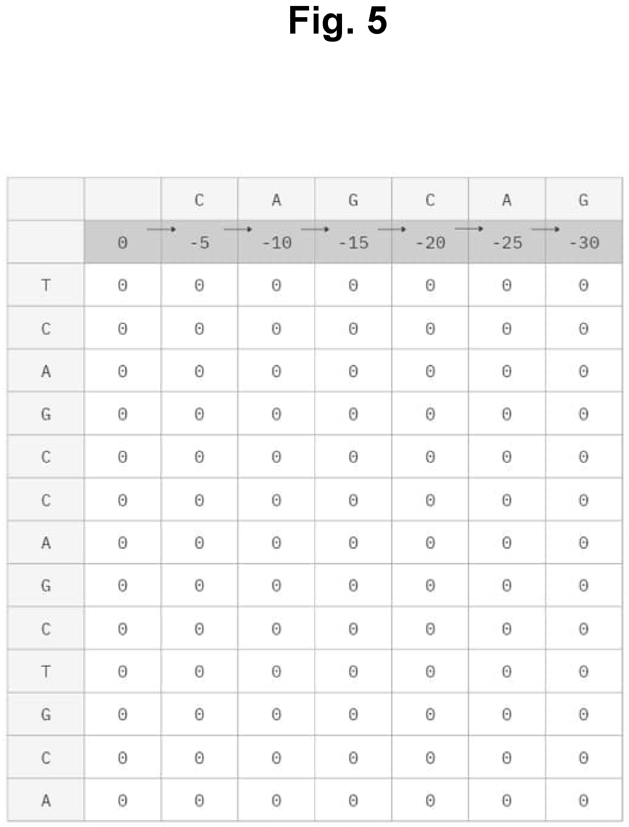

FIG. 5 shows an operation table in which operations of a target nucleic acid sequence and an oligonucleotide sequence are performed.

In FIG. 5, the target nucleic acid sequence (5'-TCAGCCAGCTGCA-3'; SEQ ID NO: 1) provided in the $13 \times 1$ matrix can be identified in the leftmost column, and the oligonucleotide sequence (5'-CAGCAG-3'; SEQ ID NO: 2) provided in the $1 \times 6$ matrix can be identified in the topmost row.

According to an embodiment of the present invention, the base includes a degenerate base and/or a universal base.

Step (b): <u>providing an operation table (**220**)</u>

The method of the present invention then comprises (b) providing an operation table. The operation table comprises a part where operation is performed and a part where a gap score is input, the part where operation is performed comprises elements ($C_{i,j}$, $1 \leq i \leq m$, $1 \leq j \leq n$) of the $m \times n$ matrix obtained by multiplying the $m \times 1$ matrix and the $1 \times n$ matrix, and the part where a gap score is input comprises an origin point ($C_{0,0}$), 0 row elements ($C_{0,j}$, $1 \leq j \leq n$), and 0 column elements ($C_{i,0}$, $1 \leq i \leq m$).

FIG. 5 shows an operation table in which operations of a target nucleic acid sequence and an oligonucleotide sequence are performed. The topmost row of the operation table describes an oligonucleotide sequence comprising 6(n) nucleotides, which indicates that the oligonucleotide sequence is provided in a $1 \times 6$(n) matrix on the operation table. In the leftmost column of the operation table, a target nucleic acid sequence comprising 13 (m) nucleotides is described, indicating that the target nucleic acid sequence is provided in a 13 (m) $\times$ 1 matrix on the operation table.

In FIG. 5, a row immediately below a row in which an oligonucleotide sequence is described and a column immediately right of a column in which a target nucleic acid sequence is described correspond to a part in which a gap score is input in an operation table. In addition, in the operation table of FIG. 5, the remaining part except for the part where the sequence is input and the part where the gap score is input is a part where the calculation is performed, and the part where the calculation is performed is a part corresponding to a column of the oligonucleotide sequence and a row of the target nucleic acid sequence.

In FIG. 5, the part where the operation is performed includes the components ($C_{i,j}$, $1 \leq i \leq 13$, and $1 \leq j \leq 6$) of the $13 \times 6$ matrix by multiplying the $13 \times 1$ matrix and the $1 \times 6$ matrix, and the part where the gap score is input includes the origin point $C_{0,0}$, 0 row components ($C_{0,j}$, and $1 \leq j \leq 6$), and 0 column components ($C_{i,0}$, and $1 \leq i \leq 13$).

Step (c): <u>inputting a gap score into the part where a gap score is input of the operation table (230)</u>

Then, the method of the present invention includes (c) inputting a gap score into the part where a gap score is input of the operation table. An origin point value + (j x gap score (k)) ($1 \leq j \leq n$, k is an integer) is input for each column element in the 0 row elements of the part where a gap score is input, and origin point value + (i x gap score (k)) ($1 \leq i \leq n$, k is an integer) is input for each row element in the 0 column elements of the part where a gap score is input.

According to an embodiment of the present invention, the origin point value included in the gap score is an integer as a value of the origin point ($C_{0,0}$), specifically, an integer selected from -20 to 20, more specifically, an integer selected from -10 to 10, still more specifically, an integer selected from -5 to 5, and most specifically, 0.

The gap score is a score assigned to consider a gap in the aligned sequence, and the degree to which the gap is assigned to the sequence may vary depending on the size of the gap score. Specifically, the gap score k is an integer selected from -20 to 20, more specifically an integer selected from -10 to 10, still more specifically an integer selected from -5 to 5, and most specifically an integer selected from -5 to 0.

FIG. 5 illustrates a process of inputting a gap score to a part where the gap score is input in the operation table. In FIG. 5, the origin point value is 0, the gap score is reduced by -5 according to the nucleotide position of the oligonucleotide sequence in the 0 row component, and the gap score is 0 according to the nucleotide position of the target nucleic acid sequence in the 0 column component.

Step (d): <u>performing an operation and inputting an operation value into the part where an operation is performed of the</u> <u>operation table</u> **(240)**

In addition, the method of the present invention comprises (d) performing an operation and inputting an operation value into the part where an operation is performed of the operation table. The operation is performed by a method comprising the following steps for each row in order from the first row to the last row of the part where an operation is performed:

d-1) obtaining a maximum value of any one operation value of addition selected from i-1) an operation value of addition in the relationship with an above matrix element and i-2) an operation value of addition in the relationship with a left matrix element, with i-3) an operation value of addition in the relationship with a left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-2) the operation value of addition in the relationship with the left matrix element is an operation value of addition obtained by adding the gap score (k, k is an integer) to the value of the left matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with any one operation value of addition from i-1) and i-2) not selected in step d-1), respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element.

In the present invention, instead of calculating an operation value for each matrix component, i) an operation value is simultaneously obtained in a relationship with a left diagonal matrix component of a matrix component for a matrix component included in a row, and ii) an operation value is simultaneously obtained in a relationship with an upper matrix component of the matrix component for a matrix component included in the row (the order of i and ii may be changed), and then a maximum value of the operation values of i) and ii) is simultaneously obtained for the matrix component included in the row. Then, in the order from the first matrix component to the last matrix component of the row, iii) an operation value is obtained in relation to the left matrix component of the matrix component for each matrix component, and a maximum value between the operation value and the maximum value of the operation values of i) and ii) is obtained as the operation value of each matrix component. In this way, the operation values are obtained up to the matrix components included in the last row. According to the present invention, the operation value of ii) and the operation value of iii) may be changed.

Such an operation method of the present invention can provide alignment information more accurately and quickly because the operation can be performed quickly on a computer, which is the biggest feature of the present invention.

According to an embodiment of the present invention, the operation is performed by a method including the following steps for each row in order from the first row to the last row of the part where the operation is performed:

d-1) obtaining a maximum value of i-1) an operation value of addition in the relationship with an above matrix element with i-3) an operation value of addition in the relationship with a left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with i-2) an operation value of addition in the relationship with the left matrix element, respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element; wherein i-2) the operation value of addition in the relationship with the left matrix element is an operation value of addition obtained by adding the gap score (k, k is an integer) to the value of the left matrix element of each matrix element of the row of the part where an operation is performed.

According to an embodiment, the operation is performed by a method including the following steps for each row in order from the first row to the last row of the part where the operation is performed:

d-1) obtaining a maximum value of i-2) an operation value of addition in the relationship with a left matrix element with i-3) an operation value of addition in the relationship with a left diagonal matrix element of each matrix element for the row of the part where an operation is performed, and inputting the maximum value as an operation value of each matrix element; wherein i-2) the operation value of addition in the relationship with the left matrix element is an operation value of addition obtained by adding the gap score (k, k is an integer) to the value of the left matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with i-1) an operation value of addition in the relationship with an above matrix element, respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element; i-1) the operation value of addition in the relationship with the above matrix element is an operation value of addition obtained by adding the gap score (k, k is an integer) to the value of the above matrix element of each matrix element of the row of the part where an operation is performed.

According to an embodiment of the present invention, the operation is performed by a method including the following steps for each row in order from the first row to the last row of the part where an operation is performed:

d-1) i-1) obtaining an operation value of addition by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element; wherein the matrix operation is a multiplication of the $m \times 1$ matrix and the $1 \times n$ matrix, and an AND operation of 4 bits of the nucleotide that is an element of the $m \times 1$ matrix and 4 bits of the nucleotide that is an element of the $1 \times n$ matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score;

i-2) obtaining an operation value of addition by adding the gap score (k, k is an integer) to the value of the above matrix element of each matrix element of the row of the part where an operation is performed;

i-3) obtaining a maximum value of an operation value of addition obtained in step i-1) and an operation value of addition obtained in step i-2) of each matrix element for the row of the part where the operation is performed, and inputting the maximum value as an operation value of each matrix element; and

d-2) obtaining the maximum value of the operation value of each matrix element input in i-3) of step d-1) with the operation value of addition obtained by adding a gap score (k) (k is an integer) to a value of the left matrix element of each matrix element of the row of the part where an operation is performed, respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element.

According to more another embodiment of the present invention, in step d-1), step i-1) and step i-2) may perform step i-1) and then step i-2), step i-2) and then step i-1), or step i-1) and step i-2) simultaneously.

According to another embodiment, the operation is performed by a method including the following steps for each row in order from the first row to the last row of the part where an operation is performed:

d-1) i-1) obtaining an operation value of addition by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element; wherein the matrix operation is a multiplication of the $m \times 1$ matrix and the $1 \times n$ matrix, and an AND operation of 4 bits of the nucleotide that is an element of the $m \times 1$ matrix and 4 bits of the nucleotide that is an element of the $1 \times n$ matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score;

i-2) obtaining an operation value of addition by adding the gap score (k, k is an integer) to the value of the left matrix element of each matrix element of the row of the part where an operation is performed;

i-3) obtaining a maximum value of an operation value of addition obtained in step i-1) and an operation value of addition obtained in step i-2) of each matrix element for the row of the part where the operation is performed, and inputting the maximum value as an operation value of each matrix element; and

d-2) obtaining the maximum value of the operation value of each matrix element input in i-3) of step d-1) with the operation value of addition obtained by adding a gap score (k) (k is an integer) to a value of the above matrix element of each matrix element of the row of the part where an operation is performed, respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element.

According to more another embodiment, in step d-1), step i-1) and step i-2) may perform step i-1) and then step i-2), step i-2) and then step i-1), or step i-1) and step i-2) simultaneously.

Since a process of obtaining a match value or a mismatch value between nucleotides of the target nucleic acid sequence and the oligonucleotide sequence by performing a matrix operation of the oligonucleotide sequence on the target nucleic acid sequence is the same as step (b) of the first aspect of the present invention, the common contents among them are omitted in order to avoid excessive complexity of the present specification due to repeated descriptions.

According to an embodiment of the present invention, the predetermined match score and the mismatch score are integers. Specifically, the predetermined match score is an integer selected from -20 to 20, more specifically an integer selected from -10 to 10, and still more specifically an integer selected from -5 to 5.

In addition, the predetermined mismatch score is specifically an integer selected from -20 to 20, more specifically an integer selected from -10 to 10, and still more specifically an integer selected from -5 to 5.

In FIG. 6, the predetermined match score is 1, and the predetermined mismatch score is -1.

According to an embodiment of the present invention, the predetermined match score and the mismatch score are the same as or different from each other. More specifically, the predetermined match score and the mismatch score are different from each other. For example, when the match score is set to 1, the mismatch score may be set to -1, and vice versa.

According to an embodiment of the present invention, when the matrix operation value is same as 4 bits of the nucleotide of the target nucleic acid sequence among the nucleotides on which the matrix operation is performed, the nucleotides on which the matrix operation is performed represent being a match, and the match value is a value expressing the match as a predetermined match score.

According to an embodiment, when the matrix operation value is same as 4 bits of the nucleotide of the oligonucleotide sequence among the nucleotides on which the matrix operation is performed, the nucleotides on which the matrix operation is performed represent being a match, and the match value is a value expressing the match as a predetermined match score.

According to an embodiment of the present invention, the method further comprises that when the matrix operation value is different from 4 bits of the nucleotide of the target nucleic acid sequence among the nucleotides on which the matrix operation is performed, the nucleotides on which the matrix operation is performed represent being a mismatch, and the mismatch value is a value expressing the mismatch as a predetermined mismatch score.

According to an embodiment, the step (d) further includes that when the matrix operation value is same as 4 bits of the nucleotide of the target nucleic acid sequence among the nucleotides on which the matrix operation is performed, the nucleotides on which the matrix operation is performed represent being a match, and the match value is a value expressing the match as a predetermined match score, and when the matrix operation value is different from 4 bits of all of the nucleotides on which the matrix operation is performed or the matrix operation value is different from 4 bits of the nucleotide of the target nucleic acid sequence on which the matrix operation is performed, the nucleotides on which the matrix operation is performed represent being a mismatch, and the mismatch value is a value expressing the mismatch as a predetermined mismatch score.

FIG. 6 illustrates a process of performing a matrix operation of a first nucleotide (base T) of a target nucleic acid sequence and an oligonucleotide sequence with respect to a first row of a part in which an operation is performed to obtain a match value or a mismatch value between nucleotides of the target nucleic acid sequence and the oligonucleotide sequence for each matrix component, and obtaining a match value or a mismatch value for each matrix component, and a value of a left diagonal matrix component of each matrix component and an operation value of addition. In addition, this process occurs simultaneously in each matrix component of the first row of the part in which the operation is performed.

In FIG. 6, the part in which the operation is performed includes the components ($C_{i,j}$, $1 \leq i \leq m$, $1 \leq j \leq n$, and m are 13 and n is 6) of the $m \times n$ matrix by multiplication of the $m \times 1$ matrix and the $1 \times n$ matrix. In FIG. 6, the predetermined match score is 1, and the predetermined mismatch score is -1.

In FIG. 6, for the first row of the portion where the operation is performed, the operation values of each matrix element are simultaneously obtained by performing the operation at the same time. The most important feature of the present invention is that the operation values of each matrix element are simultaneously obtained with respect to the row of the part in which the operation is performed, and the operation speed is increased through the operation method, thereby more quickly providing alignment information of the oligonucleotide with respect to the target nucleic acid sequence.

Although the operation values of each matrix element are obtained simultaneously, for convenience of description, each matrix element for the first row of the part in which the operation is performed will be described.

In FIG. 6, the element $(C_{1,1})$ of the first row and the first column of the elements of the $13\times6$ matrix represents a matrix operation value of 0000 by a matrix operation (multiplication of the matrix and bit And operation) of base T (0001) of the element $(A_{1,1})$ of the $13\times1$ matrix and base C (0010) of the element $(B_{1,1})$ of the $1\times6$ matrix, and the matrix operation value of 0000 is different from 4 bits 0001 of base T of the element $(A_{1,1})$ of the $13\times1$ matrix, and 4 bits 0010 of base C of the element $(B_{1,1})$ of the $1\times6$ matrix. And thus the base of the element $(A_{1,1})$ of the $13\times1$ matrix and the base of the element $(B_{1,1})$ of the $1\times6$ matrix indicates mismatch, and -1 is given as the mismatch value at this time. In addition, -1 is obtained as an addition operation value of a mismatch value (-1) of the element $(C_{1,1})$ of the first row and the first column and a value (0) of the origin point $(C_{0,0})$ which is a left diagonal matrix element of the element $(C_{1,1})$ of the first row and the first column.

In FIG. 6, the component $(C_{1,2})$ of the first row and second column of the components of the $13\times6$ matrix represents a matrix operation value of 0000 by a matrix operation (multiplication of the matrix and bit And operation) of base T (0001) of the component $(A_{1,1})$ of the $13\times1$ matrix and base A (1000) of the component $(B_{1,2})$ of the $1\times6$ matrix, and the matrix operation value of 0000 is different from 4 bits 0001 of base T of the element $(A_{1,1})$ of the $13\times1$ matrix, and 4 bits 1000 of base A of the element $(B_{1,2})$ of the $1\times6$ matrix. And thus the base of the element $(A_{1,1})$ of the $13\times1$ matrix and the base of the element $(B_{1,2})$ of the $1\times6$ matrix indicates mismatch, and -1 is given as the mismatch value at this time. In addition, -6 is obtained as an addition operation value of a mismatch value (-1) of the element $(C_{1,2})$ of the first row and the second column and a value (-5) of the left diagonal matrix element $(C_{0,1})$ of the element $(C_{1,2})$ of the first row and the second column. In this way, the operation values of the matrix components $C_{1,3}$, $C_{1,4}$, $C_{1,5}$, and $C_{1,6}$ of the first row of the part where the operation is performed are obtained as -11, -16, -21, and -26, respectively.

In FIG. 6, an arrow indicates that an operation between each matrix element and a left diagonal matrix element of each matrix element is performed.

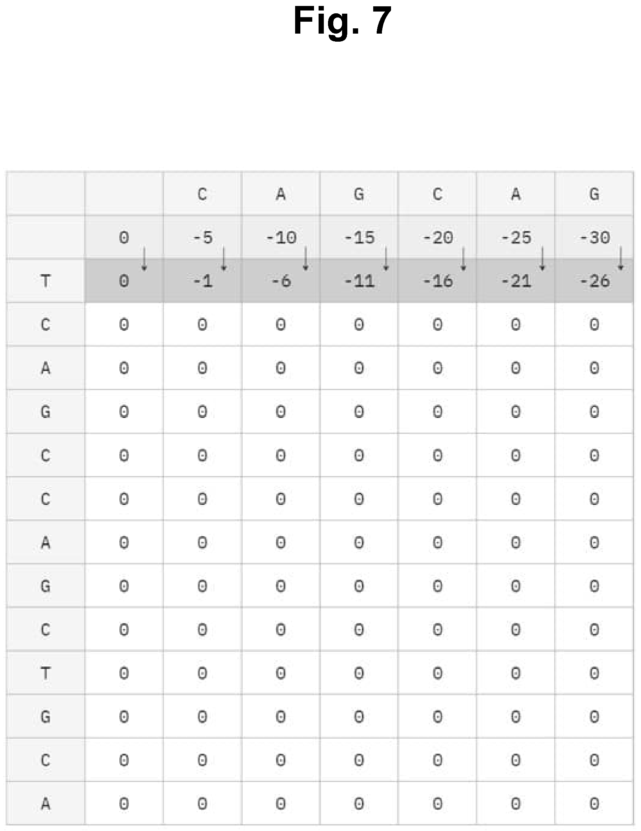

FIG. 7 shows a process of obtaining an operation value of addition in which a gap score (-5) is added to the value of the matrix component above each matrix component of the first row of the part in which the operation is performed, then obtaining a maximum value of the operation value of addition obtained in FIG. 6 of each matrix component and the operation value of addition in which a gap score (-5) is added to the value of the matrix component above each matrix component for the row of the part in which the operation is performed, and obtaining the maximum value as the operation value of each matrix component.

In FIG. 7, the element $C_{1,1}$ of the first row and the first column of the first row of the part where the operation is performed obtains -1 as the operation value, which is the maximum value of the operation value of -1 obtained in FIG. 6 and the operation value -10 of the addition obtained by adding the gap score (-5) to the value (-5) of the matrix component $C_{0,1}$ above the component $C_{1,1}$ of the first row and the first column. In addition, the component $(C_{1,2})$ of row 1 and column 2 obtains -6, which is the maximum value of the operation value of -6 obtained in FIG. 6 and the operation value (-15) of the addition obtained by adding the gap score (-5) to the operation value (-10) of the matrix component $(C_{0,2})$ above the component $(C_{1,2})$ of row 1 and column 2, as the operation value. In this way, the operation values of the matrix components $C_{1,3}$, $C_{1,4}$, $C_{1,5}$, and $C_{1,6}$ of the first row of the part where the operation is performed are obtained as -11, -16, -21, and -26, respectively.

In FIG. 7, an arrow indicates that an operation between each matrix element and the above matrix element of each matrix element is performed. However, although an arrow is displayed in the first column, the operation is not performed in the first column.

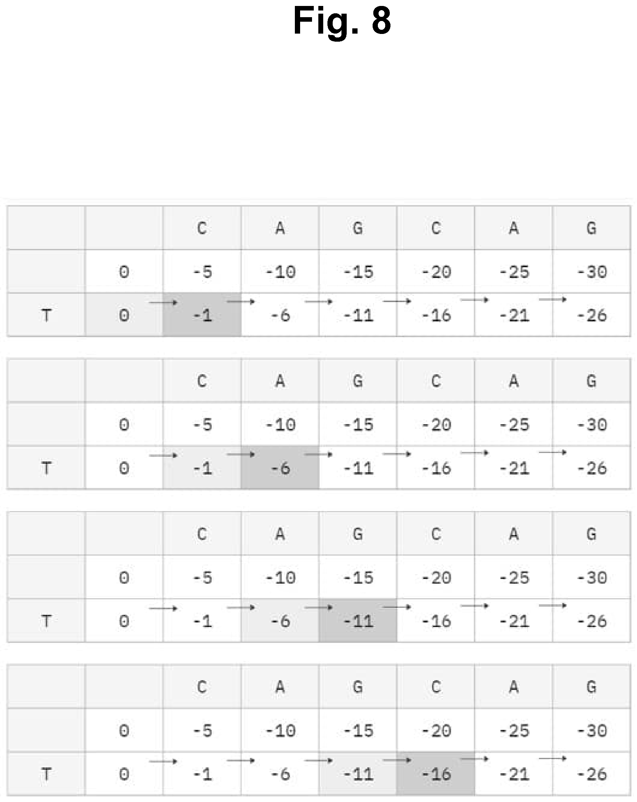

FIG. 8 illustrates a process of obtaining maximum values of the operation value of each matrix component obtained in FIG. 7 and the operation value of addition obtained by adding a gap score (-5) to the value of left matrix component of the each matrix component in the order from the first matrix component to the last matrix component of the first row of the part where the operation is performed, respectively, inputting the maximum values as operation values of each matrix component of the part where the operation is performed and displaying that the input operation value is a value originated from which matrix component among the upper, left diagonal, and left matrix components of the corresponding matrix component.

In FIG. 8, for the element $C_{1,1}$ of the first row and column, which is the first matrix component of the part where the operation is performed, the operation value of -1 is obtained, which is the maximum value of the operation value of -1 obtained in FIG. 7 and the operation value of addition of -5 obtained by adding the gap score (-5) to the value (0) of the left matrix component $C_{1,0}$ of the component $C_{1,1}$ of the first row and column, and is input as the operation value, and the input operation value is expressed as being derived from the operation value with the left diagonal matrix component $C_{0,0}$ (the indication is not shown in FIG. 8). The indication may be indicated by an arrow, for example, as an arrow in FIG. 6.

In addition, as operation value of the element $C_{1,2}$ of the first row and the second column, -6 is obtained, which is the maximum value of the operation value of -6 obtained in FIG. 7 and the operation value of addition of -6 obtained by adding the gap score (-5) to the value (-1) of the left matrix component $C_{1,1}$ of the component $C_{1,2}$ of the first row and second column, and is input as the operation value, and the input operation value is expressed as being derived from the operation value with the left diagonal matrix component $C_{0,1}$ and the operation value with the left matrix component $C_{1,1}$ (the indication is not shown in FIG. 8). Even if there are two or more origins of the input operation value, all are displayed. However, in backtracking, which will be described later, any one of them may be selected and backtracked. In this way, the operation values of the matrix components $C_{1,3}$, $C_{1,4}$, $C_{1,5}$, and $C_{1,6}$ of the first row of the part where the operation is performed are obtained and input as -11, -16, -21, and -26, respectively, and the origin is indicated.

In FIG. 8, an arrow indicates that an operation between each matrix component and a left matrix component of each matrix component is performed.

The process of obtaining the operation value of each matrix element in the second row of the part where the operation is performed is as follows: In the case of $C_{2,1}$, since the base C of the target nucleic acid sequence and the base C of the oligonucleotide sequence are matched, they have 1 as a match value, and 1 is obtained as an operation value of addition of the match value and the value (0) of $C_{1,0}$ which is a left diagonal matrix component. Then, the operation value (-6) of addition obtained by adding the gap score (-5) to the value (-1) of $C_{1,1}$, which is the above matrix component of $C_{2,1}$, is obtained, and 1 is obtained as the maximum value of the operation value (1) with the left diagonal matrix component. In this way, the operation values of the matrix components $C_{2,2}$, $C_{2,3}$, $C_{2,4}$, $C_{2,5}$ and $C_{2,6}$ of the second row of the part where the operation is performed are obtained simultaneously with the operation value of $C_{2,1}$.

Then, from the first matrix component to the last matrix component of the second row, an operation value is obtained in the relationship with the left matrix component of each matrix component, and the origin of the operation value is indicated. Specifically, in the case of $C_{2,1}$, an operation value (-5) of addition obtained by adding a gap score (-5) to a value (-0) of a left matrix component $C_{2,0}$ is obtained, a maximum value (-5) obtained from a maximum value (1) obtained from a relationship between a left diagonal matrix component and an upper matrix component of $C_{2,1}$ and the operation value(-5) obtained from a relationship with the left matrix component are obtained, input as an operation value, and indicate that it is derived from the left diagonal matrix component. Then, in this way, the operation values of the matrix components $C_{2,2}$, $C_{2,3}$, $C_{2,4}$, $C_{2,5}$, and $C_{2,6}$ of the second row of the part where the operation is performed are obtained in order, and their origins are indicated.

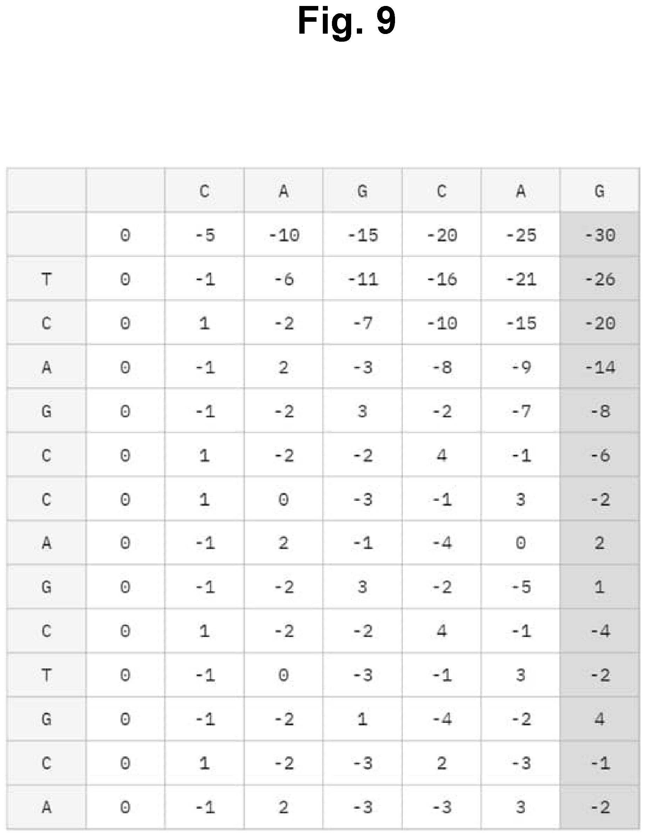

FIG. 9 shows that operation values of all matrix elements are input by performing an operation on each row of a part in which the operation is performed by the above-described method.

Step (e): performing backtracking according to the indication in step d-2) from the matrix element that satisfies a predetermined score threshold value (**250**)

Then, the method of the present invention comprises (e) performing backtracking according to the indication in step d-2) from the matrix element that satisfies a predetermined score threshold value among the operation values of the matrix elements in the last column of the part where an operation is performed where the operation value is input.

According to an embodiment of the present invention, in step (e), satisfying a predetermined score threshold value among the operation values represents that the operation value is greater than or equal to a predetermined score threshold value.

The predetermined score threshold value may be arbitrarily selected or calculated and determined through a mathematical formula. The predetermined score threshold value is, for example, an integer selected from -100 to 100.

According to an embodiment, the predetermined score threshold value is calculated by the following equation 1:

**Equation 1**

Score threshold value = (mismatch threshold value x mismatch score) + ((length of oligonucleotide - mismatch threshold value) x match score)

In the present specification, the score threshold value is a value indicating the degree of matching between the target nucleic acid sequence and the nucleotides of the oligonucleotide to be aligned.

The length of the oligonucleotide, the mismatch threshold value, the mismatch score, and the match score for calculating the score threshold value in Equation 1, are values known and are values set before performing the method of the present invention.

The mismatch threshold value indicates the threshold value at which several or fewer nucleotides in an oligonucleotide are mismatched, and for example, if the mismatch threshold value is 2, it indicates that there are two

**EP 4 752 885 A1**

or less mismatches between nucleotides of the aligned target nucleic acid sequence and the oligonucleotide. The mismatch threshold value may be selected from 0 to 60.

The match score indicates a score given to a matched nucleotide between nucleotides of the aligned target nucleic acid sequence and of the oligonucleotide, and the match score may be expressed as an integer. Specifically, the match score is an integer selected from -20 to 20, more specifically an integer selected from -10 to 10, and still more specifically an integer selected from -5 to 5.

The mismatch score indicates a score assigned to a mismatch nucleotide between nucleotides between the aligned target nucleic acid sequence and the oligonucleotide, and the mismatch score may be expressed as an integer. The mismatch score is specifically an integer selected from -20 to 20, more specifically an integer selected from -10 to 10, and still more specifically an integer selected from -5 to 5.

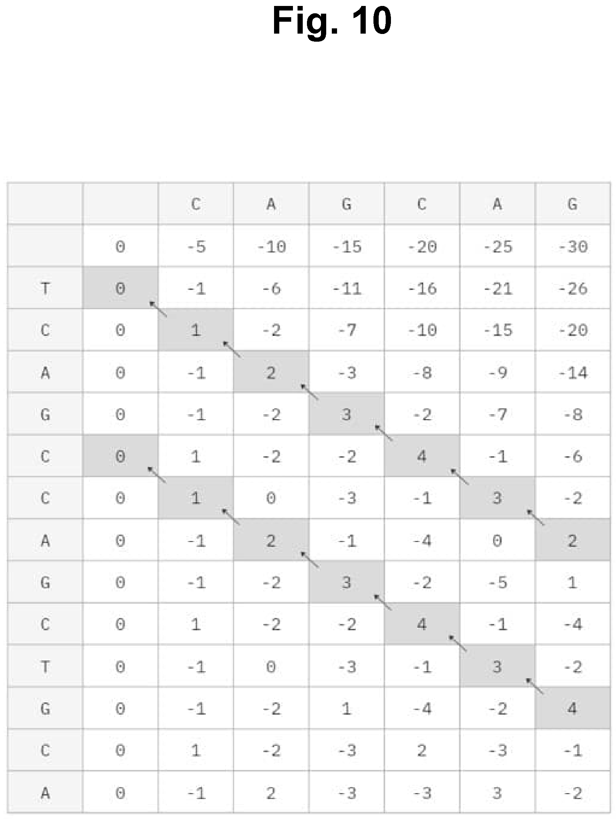

FIG. 10 illustrates a process of backtracking according to an indication from a matrix element satisfying a predetermined score threshold value among the operation values of the matrix element of the last column of the part where the operation is performed in which the operation is input.

In FIG. 10, when the mismatch threshold value 2, the mismatch score -1, the oligonucleotide length 6, and the match score 1 are set, the score threshold value according to Equation 1 is 2. In addition, among the matrix elements of the last column (j=6) of FIG. 10, the matrix elements satisfying the score threshold value 2 are $C_{7,6}$ (2) and $C_{11,6}$ (4), and as a result of backtracking according to the indication of the origin of the operation value from the matrix elements, the matrix components are traced back from $C_{7,6}$ to $C_{2,1}$ in the left diagonal matrix element direction, and traced back from $C_{11,6}$ to $C_{6,1}$ in the left diagonal matrix element direction. In FIG. 10, $C_{1,0}$ and $C_{5,0}$ are shown as a result of backtracking, but this is meaningless in alignment information.

Step (f): providing alignment information between nucleotides of the target nucleic acid sequence and nucleotides of the oligonucleotide sequence **(260)**

Finally, the method of the present invention comprises (f) providing alignment information between nucleotides of the target nucleic acid sequence and nucleotides of the oligonucleotide sequence according to the origin of the operation value of the backtracked matrix element. The alignment information comprises information selected from the group consisting of a match, a mismatch, and/or a gap between nucleotides of the target nucleic acid sequence and the oligonucleotide sequence, an alignment pattern comprising the match, mismatch, and/or gap information, the number of the mismatch, and the position of the oligonucleotide sequence in aligned target nucleic acid sequence.

According to an embodiment of the present invention, (i) when the operation value is originated from the left diagonal matrix element of the matrix element, the alignment information comprises match or mismatch information of the nucleotide of the target nucleic acid sequence of the row and the nucleotide of the oligonucleotide sequence of the column corresponding to the matrix element, (ii) when the operation value is originated from the left matrix element of the matrix element, the alignment information comprises gap information between the nucleotide of the target nucleic acid sequence of the row and the nucleotide of the target nucleic acid sequence of the next row of the matrix element, and (iii) when the operation value is originated from the above matrix element of the matrix element, the alignment information comprises gap information between the nucleotide of the oligonucleotide sequence of the column and the nucleotide of the oligonucleotide sequence of the next column of the matrix element.

FIG. 10 shows a process of providing alignment information of nucleotides of a target nucleic acid sequence and nucleotides of an oligonucleotide sequence according to the origin of an operation value of a backtracked matrix component, and the results are summarized in Table 2 below: Since the origin of the operation value is a left diagonal matrix component of the matrix component, the alignment information includes match or mismatch information of nucleotides of a target nucleic acid sequence in a row and nucleotides of an oligonucleotide sequence in a column corresponding to the matrix component.

Table 2

| Patterns | Match type | SEQ ID NO |
|---|---|---|
| CAGCAG<br>\| \| \| \|XX<br>TCAGCCAGCTGCA | 2\|N | SEQ ID NO:2<br><br>SEQ ID NO:1 |

26

(continued)

| Patterns | Match type | SEQ ID NO |
|---|---|---|
| CAGCAG<br>\| \| \| \|X\|<br>TCAGCCAGCTGCA | 1\|N | SEQ ID NO:2<br><br>SEQ ID NO:1 |

In this way, it is possible to provide semi-global alignment information of the oligonucleotide to the target nucleic acid sequence in consideration of the gap.

In the present specification, "providing alignment information" includes displaying alignment information on an output device (*e.g.,* a user terminal), allowing users to download the alignment information to a storage medium, or establishing or building the alignment information into a database in a computer.

Since the description of the local alignment, global alignment, and semi-global alignment described in the method of the first aspect of the present invention is equally applied to the method of the second aspect of the present invention, the common descriptions among them are omitted in order to avoid undue redundancy leading to the complexity of this specification.

According to an embodiment of the present invention, the method provides coverage of oligonucleotides for a plurality of target nucleic acid sequences.

By performing the method of the present invention on each of a plurality of target nucleic acid sequences, it is possible to provide the coverage of oligonucleotides for the plurality of target nucleic acid sequences.

Since the description of the plurality of target nucleic acid sequences, nucleotide databases, and coverage described in the method of the first aspect of the present invention is equally applied to the method of the second aspect of the present invention, the common descriptions among them are omitted in order to avoid undue redundancy leading to the complexity of this specification.

**Recording medium, device, and program**

In another aspect of the present invention, there is provided a computer readable storage medium containing instructions to configure a processor to perform a method for providing alignment information of an oligonucleotide for a target nucleic acid sequence, the method comprising:

(a) providing a target nucleic acid sequence comprising m nucleotides and an oligonucleotide sequence comprising n nucleotides; wherein the target nucleic acid sequence is provided as a m $\times$ 1 matrix comprising m nucleotides as matrix elements, the oligonucleotide sequence is provided as a 1 $\times$ n matrix comprising n nucleotides as matrix elements, and the nucleotides are expressed in 4 bits for each base type,

(b) providing an operation table; wherein the operation table comprises a part where operation is performed and a part where a gap score is input, the part where operation is performed comprises elements ($C_{i,j}$, 1≤i≤m, ) of the m$\times$n matrix obtained by multiplying the m$\times$1 matrix and the 1$\times$n matrix, and the part where a gap score is input comprises an origin point ($C_{0,0}$), 0 row elements ($C_{0,j}$, 1≤j≤n), and 0 column elements ($C_{i,0}$, 1≤i≤m),

(c) inputting a gap score into the part where a gap score is input of the operation table; wherein origin point value + (j x gap score (k)) (1≤j≤n, k is an integer) is input for each column element in the 0 row elements of the part where a gap score is input, and origin point value + (i x gap score (k)) (1≤i≤n, k is an integer) is input for each row element in the 0 column elements of the part where a gap score is input,

(d) performing an operation and inputting an operation value into the part where an operation is performed of the operation table; wherein the operation is performed by a method comprising the following steps for each row in order from the first row to the last row of the part where an operation is performed,

d-1) obtaining a maximum value of any one operation value of addition selected from i-1) an operation value of addition in the relationship with an above matrix element and i-2) an operation value of addition in the relationship with a left matrix element, with i-3) an operation value of addition in the relationship with a left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-2) the operation value of addition in the relationship with the left matrix element is an operation value of addition obtained by adding the gap score (k, k is an integer) to the value of the left matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the $m \times 1$ matrix and the $1 \times n$ matrix, and an AND operation of 4 bits of the nucleotide that is an element of the $m \times 1$ matrix and 4 bits of the nucleotide that is an element of the $1 \times n$ matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with any one operation value of addition from i-1) and i-2) not selected in step d-1), respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element;

(e) performing backtracking according to the indication in step d-2) from the matrix element that satisfies a predetermined score threshold value among the operation values of the matrix elements in the last column of the part where an operation is performed where the operation value is input; and

(f) providing alignment information between nucleotides of the target nucleic acid sequence and nucleotides of the oligonucleotide sequence according to the origin of the operation value of the backtracked matrix element; wherein the alignment information comprises information selected from the group consisting of a match, a mismatch, and/or a gap between nucleotides of the target nucleic acid sequence and the oligonucleotide sequence, an alignment pattern comprising the match, mismatch, and/or gap information, the number of the mismatch, and the position of the oligonucleotide sequence in aligned target nucleic acid sequence.

In still another aspect of the present invention, there is provided a computer program to be stored on a computer readable storage medium, to configure a processor to perform a method for providing alignment information of an oligonucleotide for a target nucleic acid sequence, the method comprising:

(a) providing a target nucleic acid sequence comprising m nucleotides and an oligonucleotide sequence comprising n nucleotides; wherein the target nucleic acid sequence is provided as a $m \times 1$ matrix comprising m nucleotides as matrix elements, the oligonucleotide sequence is provided as a $1 \times n$ matrix comprising n nucleotides as matrix elements, and the nucleotides are expressed in 4 bits for each base type,

(b) providing an operation table; wherein the operation table comprises a part where operation is performed and a part where a gap score is input, the part where operation is performed comprises elements ($C_{i,j}$, $1 \leq i \leq m$, $1 \leq j \leq n$) of the $m \times n$ matrix obtained by multiplying the $m \times 1$ matrix and the $1 \times n$ matrix, and the part where a gap score is input comprises an origin point ($C_{0,0}$), 0 row elements ($C_{0,j}$, $1 \leq j \leq n$), and 0 column elements ($C_{i,0}$, $1 \leq i \leq m$),

(c) inputting a gap score into the part where a gap score is input of the operation table; wherein origin point value + (j x gap score (k)) ($1 \leq j \leq n$, k is an integer) is input for each column element in the 0 row elements of the part where a gap score is input, and origin point value + (i x gap score (k)) ($1 \leq i \leq n$, k is an integer) is input for each row element in the 0 column elements of the part where a gap score is input,

(d) performing an operation and inputting an operation value into the part where an operation is performed of the operation table; wherein the operation is performed by a method comprising the following steps for each row in order from the first row to the last row of the part where an operation is performed,

d-1) obtaining a maximum value of any one operation value of addition selected from i-1) an operation value of addition in the relationship with an above matrix element and i-2) an operation value of addition in the relationship with a left matrix element, with i-3) an operation value of addition in the relationship with a left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-2) the operation value of addition in the relationship with the left matrix element is an operation value of addition obtained by adding the gap score (k, k is an integer) to the value of the left matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the $m \times 1$ matrix and the $1 \times n$ matrix, and an AND operation of 4 bits of the nucleotide that is an element of the $m \times 1$ matrix and 4 bits of the nucleotide that is an element of the $1 \times n$ matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with any one operation value of addition from i-1) and i-2) not selected in step d-1), respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element;

(e) performing backtracking according to the indication in step d-2) from the matrix element that satisfies a predetermined score threshold value among the operation values of the matrix elements in the last column of the part where an operation is performed where the operation value is input; and

(f) providing alignment information between nucleotides of the target nucleic acid sequence and nucleotides of the oligonucleotide sequence according to the origin of the operation value of the backtracked matrix element; wherein the alignment information comprises information selected from the group consisting of a match, a mismatch, and/or a gap between nucleotides of the target nucleic acid sequence and the oligonucleotide sequence, an alignment pattern comprising the match, mismatch, and/or gap information, the number of the mismatch, and the position of the oligonucleotide sequence in aligned target nucleic acid sequence.

In another aspect of the present invention, there is provided a device for providing alignment information of an oligonucleotide relative to a target nucleic acid sequence, comprising (a) a computer processor and (b) a computer readable storage medium of the present invention coupled to the computer processor.

Since the storage medium, the device, and the computer program of the prevent invention are intended to perform the present methods described as above in a computer, the common descriptions among them are omitted in order to avoid undue redundancy leading to the complexity of this specification.

The program instructions are operative, when performed by the processor, to cause the processor to perform the present method described above. The program instructions for performing a method for providing alignment information of an oligonucleotide for a target nucleic acid sequence may comprise the following instructions: (i) an instruction to provide a target nucleic acid sequence comprising m nucleotides and an oligonucleotide sequence comprising n nucleotides; (ii) an instruction to provide an operation table; (iii) an instruction to input a gap score into the part where a gap score is input of the operation table; (iv) an instruction to perform an operation and input an operation value into the part where an operation is performed of the operation table; (v) an instruction to perform backtracking according to the indication from the matrix element that satisfies a predetermined score threshold value among the operation values of the matrix elements in the last column of the part where an operation is performed where the operation value is input; and (vi) an instruction to provide (e.g., to display on an output device) alignment information between nucleotides of the target nucleic acid sequence and nucleotides of the oligonucleotide sequence according to the origin of the operation value of the backtracked matrix element.

Since the description of the processor, the storage medium, the method of providing alignment information, the instructions for implementing the processor, and the computer processor described in the first aspect of the present invention are equally applied to the second aspect of the present invention, the common descriptions among them are omitted in order to avoid undue redundancy leading to the complexity of this specification.

**EFFECTS OF THE INVENTION**

The features and advantages of the present invention are summarized as follows:

(a) In the related art, when providing alignment information or coverage of oligonucleotides using a local alignment (specifically, BLAST) with respect to a nucleic acid sequence itself, alignment information or coverage for a portion of the oligonucleotide sequence (partial length) was provided due to the nature of local alignment that checks for local mismatches. Alternatively, in order to overcome this problem, a program including a repair method is required, but even when the repair method is used, when the number of nucleic acid sequences is very large, there was a problem in that a match or mismatch pattern for the entire oligonucleotide sequence (the entire length) could not be shown due to a failure of a memory or the like.

(b) The method of the present invention, which is capable of providing semi-global alignment of oligonucleotides for a target nucleic acid sequence by applying dynamic programming, can provide alignment information for the entire length of the oligonucleotide more accurately and rapidly with less computer resources than the conventional method, and can provide coverage of the oligonucleotides for a plurality of target nucleic acid sequences.

## BRIEF DESCRIPTION OF THE DRAWINGS

FIG. 1 is a flowchart of processes of performing a method of the present invention according to an embodiment of the present invention.

FIG. 2 shows a process of obtaining a match value or a mismatch value between nucleotides of a target nucleic acid sequence and an oligonucleotide sequence by performing a matrix operation of the oligonucleotide sequence on the target nucleic acid sequence in an operation table according to an embodiment of the present invention.

FIG. 3 represents a process of calculating a sum of match values and/or mismatch values of diagonal elements of a $6\times6$ square matrix by performing a diagonal sum (trace) operation of the $6\times6$ square matrix in the $13\times6$ matrix in which the matrix operation is performed according to an embodiment of the present invention.

FIG. 4 is a flowchart of processes of performing a method of the present invention according to another embodiment of the present invention.

FIG. 5 illustrates a process in which a gap score is input to a part where a gap score is input in an operation table in which an operation of a target nucleic acid sequence and an oligonucleotide sequence is performed according to another embodiment of the present invention.

FIG. 6 illustrates a process of obtaining a match value or a mismatch value between nucleotides of a target nucleic acid sequence and an oligonucleotide sequence for each matrix element by performing a matrix operation of a first nucleotide (base T) of a target nucleic acid sequence and an oligonucleotide sequence for a first row of a part in which an operation is performed, and obtaining an operation value of addition of adding the match value or the mismatch value for each matrix element and the value of a left diagonal matrix element of each matrix element, according to another embodiment of the present invention.

FIG. 7 shows a process of obtaining an operation value of addition in which a gap score (-5) is added to the value of the matrix element above each matrix element of the first row of the part in which the operation is performed, then obtaining a maximum value of the operation value of addition obtained in FIG. 6 of each matrix element and the operation value of addition in which a gap score (-5) is added to the value of the matrix element above each matrix element for the row of the part in which the operation is performed, and obtaining the maximum value as the operation value of each matrix element, according to another embodiment of the present invention.

FIG. 8 illustrates a process of obtaining maximum values of the operation value of each matrix element obtained in FIG. 7 and the operation value of addition obtained by adding a gap score (-5) to the value of left matrix component of the each matrix element in the order from the first matrix element to the last matrix element of the first row of the part where the operation is performed, respectively, inputting the maximum values as operation values of each matrix element of the part where the operation is performed and displaying that the input operation value is a value originated from which matrix element among the upper, left diagonal, and left matrix elements of the corresponding matrix element, according to another embodiment of the present invention.

FIG. 9 shows that the operation values of all matrix elements are input by performing an operation on each row of a part in which the operation is performed according to another embodiment of the present invention.

FIG. 10 illustrates a process of backtracking according to an indication from a matrix element satisfying a predetermined score threshold value among the operation values of the matrix element of the last column of the part where the operation is performed in which the operation value is input according to another embodiment of the present invention.

The present invention will now be described in further detail by examples. It would be obvious to those skilled in the art that these examples are intended to be more concretely illustrative and the scope of the present invention as set forth in the appended claims is not limited to or by the examples.

## Examples

**Comparative Example 1: Provision of alignment information and coverage of oligonucleotides for coronavirus nucleic acid sequences by using sequence coverage tool (SCT) based on BLAST**

*(1) Provision of oligonucleotides*

Primer and probe sequence information for amplification and detection of SARS-Cov-2 provided by CDC were used as oligonucleotide sequences. Sequence information is available at CDC's Influenza SARS-CoV-2 Multiplex Assay | CDC. Among the sequences provided herein, a forward primer that is SC2-F (5'-CTGCAGATTTGGATGATTTCTCC-3': SEQ ID NO:3) was used.

*(2) Provision of nucleotide database*

Sequence information of GISAID (gisaid.org) published in the SARS-CoV-2 genome sequence database was used as a nucleic acid sequence included in the nucleotide database. Sequences listed in the GISAID have collection date information, which is time information at which sequences were collected. Only sequences up to December 31, 2020 were collected from the sequences listed in the GISAID and used as nucleotide databases.

The number of the GISAID SARS-CoV-2 genome sequences collected through the above method is total 722,704 genome sequences, and the data files were stored at a size of 21,433,967,219 bytes (20 Gb), based on the FASTA file format typically used for sequence storage.

*(3) Searching in nucleic acid sequence using SCT*

The SCT internally uses BLAST to perform a pairwise local alignment with each oligonucleotide sequence and target nucleic acid sequences included in the nucleotide database. The parameters used in the SCT are shown in Table 3 below:

Table 3

| Parameter | value |
| --- | --- |
| Oligonucleotide sequence file name of Fasta type (query) | 2019-nCoV_N1-F.fa |
| Database filename consisting of the SARS-CoV-2 source genome sequence (db) | gisaid_seq. fa |
| Selection of nucleotide search task (task) | Blastn |
| Minimum Identity value to search (perc_identity) | 60% |
| The minimum length of the word size used in the blast program algorithm (word_size) | 4 |
| A sufficiently large value to include all of the information that is searched. | 100000 |
| A value large enough to include all of the information that is searched (max_target_secs) | 1000000 |
| Do not filter using string complexity of database sequences (dust) | No |
| Number of CPU cores used in the test (num_threads) | 60 |
| Output File Format Number (outfmt) | 6 |
| Output only if it covers more than 95% of the oligonucleotide length (qcov_hsp_prec) | 95 |
| Result File Name (out) | F_sub_ori. txt |

As a result of performing SCT on the nucleic acid sequences, the analysis was performed in the nucleotide database for about 225 minutes. As a result of the analysis, hit information satisfying the parameter was provided, and among them, the top four information of the alignment pattern with a large number of hits are summarized in Table 4 below.

Table 4

| Patterns | SEQ ID NO | Match type | Counts |
|---|---|---|---|
| CTGCAGATTTGGATGATTTCTCC<br><br>\|\|\|\|\|\|\|\|\|\|\|\|\|\|\|\|\|\|\|\|\|\|\|<br>CTGCAGATTTGGATGATTTCTCC | SEQ ID NO:3<br><br>SEQ ID NO:4 | 0\|N | 680,612 |
| CTGCAGATTTGGATGATTTCTCC<br><br>X\|XXX\|\|\|\|\|\|\|\|\|\|\|X\|X\|XX<br>TTTAGGATTTGGATGATCTATGT | SEQ ID NO:3<br><br>SEQ ID NO:5 | -8\|N | 25,025 |
| CTGCAGATTTGGATGATTTCTCC<br><br>\|\|\|X\|\|\|\|\|\|\|\|\|\|\|\|\|\|\|\|<br>CTGTAGATTTGGATGATTTCTCC | SEQ ID NO:3<br><br>SEQ ID NO:6 | 1\|N | 5,538 |
| CTGCAGATTTGGATGATTTCTCC<br><br>\|\|\|\|\|\|\|\|\|\|\|X\|\|\|\|\|\|\|\|\|<br>CTGCAGATTTGTATGATTTCTCC | SEQ ID NO:3<br><br>SEQ ID NO:7 | 1\|N | 3,186 |

The above output information is the result obtained by expanding the entire range of the oligonucleotide sequence after BLAST analysis, which is a local alignment.

**Example 1: Provision of alignment information and coverage of oligonucleotides for coronavirus nucleic acid sequences by using OligoSCAN without considering Gap**

*(1) Provision of oligonucleotides*

Oligonucleotides were provided in the same manner as in Comparative Example 1.

*(2) Provision of nucleotide database*

A nucleotide database was provided in the same manner as in Comparative Example 1.

*(3) Searching in nucleic acid sequence using semi-global alignment method*

OligoSCAN refers to a series of analysis pipelines (programs) composed of sequential invocations of analysis package functions, is implemented as an algorithm implemented in a python environment using bit-operation and matrixoperation, and uses an internal algorithm modified in a semi-global alignment method so that only global alignment of oligonucleotides can be considered. However, in Example 1, an OligoSCAN without considering a gap was used.

The following instructions execute the OligoSCAN analysis pipeline. The parameters are shown in Table 5 below. Table 6 shows the information included in the analysis parameter file.

Table 5

| Parameter | Value |
|---|---|
| Oligonucleotide sequence file (input_oligo_fasta) | Path to the fasta format file where SC2-F sequence information is recorded |
| Oligonucleotide information file (input_oligo_meta) | - |
| Nucleotide database sequence file (input_acc_fasta) | Path to the fasta format file where the nucleotide sequence to be used for analysis is recorded |

(continued)

| Parameter | Value |
|---|---|
| Nucleotide database information file (input_acc_meta) | - |
| Analysis parameters file | Path to the json format file where the user input information used for analysis is written. |

Table 6

| Parameter | Value |
|---|---|
| Number of CPU cores used in the test (num_threads) | 32 |
| Mismatch Threshold (mismatch_thr) | 12 |
| Partial Threshold (partial_thr) | 0 |
| N Threshold (allow_n_thr) | 2 |

When an OligoSCAN without gap consideration was performed on the nucleic acid sequences, the analysis was performed for about 39 minutes. Hit information satisfying the parameter was provided, and among them, the top four alignment pattern information having a large number of hits are summarized in Table 7 below.

Table 7

| Patterns | SEQ ID NO | Match type | Counts |
|---|---|---|---|
| CTGCAGATTTGGATGATTTCTCC<br>\|\|\|\|\|\|\|\|\|\|\|\|\|\|\|\|\|\|\|\|\|\|\|<br>CTGCAGATTTGGATGATTTCTCC | SEQ ID NO:3<br><br>SEQ ID NO:4 | 0\|N | 680,612 |
| CTGCAGATTTGGATGATTTCTCC<br>X\|X\|\|\|\|XX\|XX\|\|\|\|X\|\|\|X\|\|<br>GTACAGAAGTAAATGAGTTCGCC | SEQ ID NO:3<br><br>SEQ ID NO:8 | 8\|N | 22,175 |
| CTGCAGATTTGGATGATTTCTCC<br>\|\|\|X\|\|\|\|\|\|\|\|\|\|\|\|\|\|\|\|\|<br>CTGTAGATTTGGATGATTTCTCC | SEQ ID NO:3<br><br>SEQ ID NO:6 | 1\|N | 5,538 |
| CTGCAGATTTGGATGATTTCTCC<br>\|\|\|\|\|\|\|\|\|\|\|X\|\|\|\|\|\|\|\|\|\|<br>CTGCAGATTTGTATGATTTCTCC | SEQ ID NO:3<br><br>SEQ ID NO:7 | 1\|N | 3,186 |

In Example 1, compared to Example 2 to be described later, a pattern was generally confirmed similarly, but since gap was not included, it was confirmed that the number of mismatches was 1 to 2 higher. In addition, the coverage of the oligonucleotide may be calculated through a ratio of the number of counts of the alignment pattern satisfying the reference or threshold of a predetermined match type to the number of target nucleic acid sequences serving as the reference.

**Example 2: Provision of alignment information and coverage of oligonucleotides for coronavirus nucleic acid sequences using OligoSCAN considering Gap**

*(1) Provision of oligonucleotides*

Oligonucleotides were provided in the same manner as in Comparative Example 1.

*(2) Provision of nucleotide database*

A nucleotide database was provided in the same manner as in Comparative Example 1.

*(3) Searching in nucleic acid sequence using semi-global alignment method*

The same method as in Example 1 was performed, but in Example 2, an OligoSCAN considering a gap was used.
As a result of performing an OligoSCAN considering a gap on the nucleic acid sequences, the analysis was performed for about 45 minutes. Hit information satisfying the parameter was provided, and among them, the top four alignment pattern information having a large number of hits are summarized in Table 8 below.

Table 8

| Patterns | SEQ ID NO | Match type | Counts |
|---|---|---|---|
| CTGCAGATTTGGATGATTTCTCC<br>\|\|\|\|\|\|\|\|\|\|\|\|\|\|\|\|\|\|\|\|\|\|\|<br>CTGCAGATTTGGATGATTTCTCC | SEQ ID NO:3<br><br>SEQ ID NO:4 | 0\|N | 680,612 |
| CTGCAG–ATTTGGATGATTTCTCC<br>\|\|XX\|\|X\|\|\|\|\|\|\|\|\|X\|X\|XX<br>CTTTAGGATTTGGATGATCTATGT | SEQ ID NO:3<br><br>SEQ ID NO:9 | -7\|N | 23,030 |
| CTGCAGATTTGGATGATTTCTCC<br>\|\|\|X\|\|\|\|\|\|\|\|\|\|\|\|\|\|\|\|<br>CTGTAGATTTGGATGATTTCTCC | SEQ ID NO:3<br><br>SEQ ID NO:6 | 1\|N | 5,538 |
| CTGCAGATTTGGATGATTTCTCC<br>\|\|\|\|\|\|\|\|\|\|\|X\|\|\|\|\|\|\|\|\|\|<br>CTGCAGATTTGTATGATTTCTCC | SEQ ID NO:3<br><br>SEQ ID NO:7 | 1\|N | 3,186 |

Compared to Comparative Example 1, the analysis was performed about five times faster, and it was confirmed that the hit result found a pattern with less mismatch including a gap or found patterns that were not previously found.
In the alignment pattern indicating the match type -7|N, the horizontal bar (-) of the oligonucleotide sequence indicates gap information, and the minus (-) sign in the match type -7|N indicates that the oligonucleotide sequence as a query sequence and the target nucleic acid sequence included in the nucleotide database were checked for reverse or reverse complementary mismatch.
Meanwhile, it was confirmed that the coverage of the oligonucleotide exhibiting match type 0|N among the top four alignment patterns was 95.5%.

**Comparative Example 2: Provision of alignment information and coverage of oligonucleotides for *Bacteroides Fragilis* nucleic acid sequences using Sequence Coverage Tool (SCT) based on BLAST**

*(1) Provision of oligonucleotides*

Dual specificity oligonucleotide (Dual priming oligonucleotide; DPO) primers were used as oligonucleotides, and sequence information is as follows: DPO-F (5'-GACTCTAGAGAGACTGCCGTCGTAAIIIIIGAGGAAGGTG-3':SEQ ID NO:10, forward primer)

*(2) Provision of nucleotide database*

For Bacteroides fragilis (taxon id: 817), subtaxon sequences were collected from the GenBank database and used as a nucleotide database.

A total of 49,889 genome sequences were collected, and data files were stored at a size of 1,899,478,690 bytes (1.76 Gb), based on the FASTA file format commonly used for sequence storage.

*(3) Searching in nucleic acid sequence using SCT*

It was performed in the same manner as in Comparative Example 1. The parameters shown in Table 3 were used as they were, and only the input oligonucleotide sequence and target nucleotide sequence information were changed.

As a result of performing SCT on the nucleic acid sequences, the analysis was performed in the nucleotide database for about 10 minutes. As a result of the analysis, hit information satisfying the parameter was provided, and among them, the top four information of the alignment pattern with a large number of hits are summarized in Table 9 below.

Table 9

| Patterns | SEQ ID NO | match type | count |
|---|---|---|---|
| GACTCTAGAGAGACTGCCGTCGTAAIIIIIGAGGAAGGTG<br>\|\|\|\|\|\|X\|\|\|\|\|\|\|\|\|\|\|\|\|\|\|\|\|\|\|-----\|\|\|\|\|\|\|\|\|\|\|<br>GACTCTGGAGAGACTGCCGTCGTAAGATGTGAGGAAGGTG | SEQ ID NO:10<br><br>SEQ ID NO:11 | -1\|0 | 199 |
| GACTCTAGAGAGACTGCCGTCGTAAIIIIIGAGGAAGGTG<br>\|\|X\|\|\|\|X\|\|X\|\|X\|\|\|\|\|\|XXXXX-----XX\|X\|\|\|X\|X<br>GATTCTATAGTGATTGCCGTAAAGTCGGGATTGAAAGATA | SEQ ID NO:10<br><br>SEQ ID NO:12 | -9\|5 | 161 |
| GACTCTAGAGAGACTGCCGTCGTAAIIIIIGAGGAAGGTG<br>XXXXXXX\|\|\|\|\|\|\|\|\|\|XXXXX\|XX-----XXX\|X\|XX\|X<br>CCTCAAGGAGAGACTGCATATTTCGAAGAAAGCGCACCTC | SEQ ID NO:10<br><br>SEQ ID NO:13 | 14\|7 | 157 |
| GACTCTAGAGAGACTGCCGTCGTAAIIIIIGAGGAAGGTG<br>XX\|\|X\|\|X\|\|\|\|\|\|\|\|\|\|XXXXXX-----\|\|\|X\|X\|XXX<br>ATCTTTACAGAGACTGCCGATCAGGAAAAAGAGCATGCAA | SEQ ID NO:10<br><br>SEQ ID NO:14 | 10\|5 | 152 |

The above output information is the result obtained by expanding the entire range of the oligonucleotide sequence after BLAST analysis, which is a local alignment.

**Example 3: Provision of alignment information and coverage of oligonucleotides on *Bacteroides Fragilis* nucleic acid sequences by OligoSCAN without considering Gap**

*(1) Provision of oligonucleotides*

Oligonucleotides were provided in the same manner as in Comparative Example 2.

*(2) Provision of nucleotide database*

A nucleotide database was provided in the same manner as in Comparative Example 2.

*(3) Searching in nucleic acid sequence using semi-global alignment method*

This was performed in the same manner as in Example 1, but the changed parts are the input sequence information and the target nucleotide sequence information.

As a result of performing an OligoSCAN without considering a gap on the nucleic acid sequences, the analysis was performed for about 1 minute and 40 seconds. Hit information satisfying the parameters was provided, and the top four information of the combination pattern having a large number of hits are summarized in Table 10 below.

Table 10

| Patterns | SEQ ID NO | match type | count |
|---|---|---|---|
| GACTCTAGAGAGACTGCCGTCGTAAIIIIIGAGGAAGGTG<br>\|\|\|\|\|\|X\|\|\|\|\|\|\|\|\|\|\|\|\|\|\|\|\|\|-----\|\|\|\|\|\|\|\|\|\|<br>GACTCTGGAGAGACTGCCGTCGTAAGATGTGAGGAAGGTG | SEQ ID NO:10<br><br>SEQ ID NO:11 | -1\|0 | 199 |
| GACTCTAGAGAGACTGCCGTCGTAAIIIIIGAGGAAGGTG<br>\|\|\|\|\|\|\|\|\|\|\|\|\|\|\|\|\|\|\|\|\|\|\|\|\|-----\|\|\|\|\|\|\|\|\|\|<br>GACTCTAGAGAGACTGCCGTCGTAAGATGTGAGGAAGGTG | SEQ ID NO:10<br><br>SEQ ID NO:15 | -0\|0 | 172 |
| GACTCTAGAGAGACTGCCGTCGTAAIIIIIGAGGAAGGTG<br>\|\|X\|XX\|\|\|X\|\|\|\|\|\|X\|\|X\|XX\|\|-----\|XX\|\|\|\|XX\|<br>GAGTAAAGACAGACTGTCGGCAAAATCTTTGTAGAAGTCG | SEQ ID NO:10<br><br>SEQ ID NO:16 | 8\|4 | 164 |
| GACTCTAGAGAGACTGCCGTCGTAAIIIIIGAGGAAGGTG<br>\|\|\|\|\|\|X\|\|\|\|\|\|\|\|\|\|\|\|\|\|\|\|\|\|-----\|\|\|\|\|\|\|\|\|\|<br>GACTCTGGAGAGACTGCCGTCGTAAGATGTGAGGAAGGTG | SEQ ID NO:10<br><br>SEQ ID NO:11 | 1\|0 | 147 |

As a result of comparison with Comparative Example 2, it was confirmed that the number of good match type patterns occupied a high number. In addition, it was confirmed that the results of Example 3 were similar to those of Example 4 to be described later, and it was confirmed that the number of mismatches occurred more in some patterns due to the characteristics of the algorithm that did not consider the gap.

**Example 4: Provision of alignment information and coverage of oligonucleotides for *Bacteroides Fragilis* nucleic acid sequences using OligoSCAN considering Gap**

*(1) Provision of oligonucleotides*

Oligonucleotides were provided in the same manner as in Comparative Example 2.

*(2) Provision of nucleotide database*

A nucleotide database was provided in the same manner as in Comparative Example 2.

*(3) Searching in nucleic acid sequence using semi-global alignment method*

This was performed in the same manner as in Example 2, except that the changed parts are the input sequence information and the target nucleotide sequence information.
As a result of performing an OligoSCAN considering a gap on the nucleic acid sequences, the analysis was performed for about 1 minute and 40 seconds. Hit information satisfying the parameters was provided, and the top four information of the combination pattern having a large number of hits are summarized in Table 11 below.

Table 11

| Patterns | SEQ ID NO | match type | count |
|---|---|---|---|
| GACTCTAGAGAGACTGCCGTCGTAAIIIIIGAGGAAGGTG<br>\|\|\|\|\|\|X\|\|\|\|\|\|\|\|\|\|\|\|\|\|\|\|\|\|-----\|\|\|\|\|\|\|\|\|\|<br>GACTCTGGAGAGACTGCCGTCGTAAGATGTGAGGAAGGTG | SEQ ID NO:10<br><br>SEQ ID NO:11 | -1\|0 | 199 |

(continued)

| Patterns | SEQ ID NO | match type | count |
|---|---|---|---|
| GACTCTAGAGAGACTGCCGTCGTAAI I I I IGAGGAAGGTG<br><br>X I I I XXX I X I I I I I I XX I X I XX I I I -----I I I I I I I I I I<br><br>AACTTCGGGGGAGACTATCCTT-TAAGAAGAGAGGAAGGTG | SEQ ID NO:10<br><br>SEQ ID NO:17 | -10\|0 | 179 |
| GACTCTAGAGAGACTGCCGTCGTAAI I I I IGAGGAAGGTG<br><br>I I I I I I I I I I I I I I I I I I I I I I I I I I I I I -----I I I I I I I I I I<br><br>GACTCTAGAGAGACTGCCGTCGTAAGATGTGAGGAAGGTG | SEQ ID NO:10<br><br>SEQ ID NO:15 | -0\|0 | 172 |
| GACTCTAGAGAGACTGCCGTCGTAAI I I I IGAGGAAGGTG<br><br>I I I I I I X I I I I I I I I I I I I I I I I I I I I I -----I I I I I I I I I I<br><br>GACTCTGGAGAGACTGCCGTCGTAAGATGTGAGGAAGGTG | SEQ ID NO:10<br><br>SEQ ID NO:11 | 1\|0 | 147 |

As a result of comparison with Comparative Example 2, it was confirmed that the number of good match type patterns occupied a high number. In addition, in Comparative Example 2, when BLAST was used and the DPO primer was treated, only the front part of the inosine linker was used, and thus it was confirmed that many mismatches occurred at the rear part of the inosine linker. On the other hand, in Example 4, since alignment was performed on the entire primer sequence, it was confirmed that the number of mismatches was also low at the rear part of the inosine linker.

Having described a preferred embodiment of the present invention, it is to be understood that variants and modifications thereof falling within the spirit of the invention may become apparent to those skilled in this art, and the scope of this invention is to be determined by appended claims and their equivalents.

**Claims**

1. A computer-implemented method for providing alignment information of an oligonucleotide for a target nucleic acid sequence comprising:

(a) providing a target nucleic acid sequence comprising m nucleotides and an oligonucleotide sequence comprising n nucleotides; wherein the target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type,

(b) performing a matrix operation of the oligonucleotide sequence for the target nucleic acid sequence to obtain a match value or mismatch value between nucleotides of the target nucleic acid sequence and the oligonucleotide sequence; wherein the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when the matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score,

(c) performing a trace operation of a n × n square matrix in the m × n matrix on which the matrix operation is performed to obtain the sum of match values and/or mismatch values of diagonal elements of the n × n square matrix; and

(d) when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold value; wherein the alignment information comprises information selected from the group consisting of a match and/or mismatch between nucleotides of the target nucleic acid sequence and the oligonucleotide sequence, an alignment pattern comprising the match and/or mismatch information, the number of the mismatch, and the position of the oligonucleotide sequence in aligned target nucleic acid sequences.

2. A computer-implemented method for providing alignment information of an oligonucleotide for a target nucleic acid sequence comprising:

(a) providing a target nucleic acid sequence comprising m nucleotides and an oligonucleotide sequence comprising n nucleotides; wherein the target nucleic acid sequence is provided as a m $\times$ 1 matrix comprising m nucleotides as matrix elements, the oligonucleotide sequence is provided as a 1 $\times$ n matrix comprising n nucleotides as matrix elements, and the nucleotides are expressed in 4 bits for each base type,

(b) providing an operation table; wherein the operation table comprises a part where operation is performed and a part where a gap score is input, the part where operation is performed comprises elements ($C_{i,j}$, 1≤i≤m, 1≤j≤n) of the m$\times$n matrix obtained by multiplying the m$\times$1 matrix and the 1$\times$n matrix, and the part where a gap score is input comprises an origin point ($C_{0,0}$), 0 row elements ($C_{0,j}$, 1≤j≤n), and 0 column elements ($C_{i,0}$, 1≤i≤m),

(c) inputting a gap score into the part where a gap score is input of the operation table; wherein origin point value + (j x gap score (k)) (1≤j≤n, k is an integer) is input for each column element in the 0 row elements of the part where a gap score is input, and origin point value + (i x gap score (k)) (1≤i≤n, k is an integer) is input for each row element in the 0 column elements of the part where a gap score is input,

(d) performing an operation and inputting an operation value into the part where an operation is performed of the operation table; wherein the operation is performed by a method comprising the following steps for each row in order from the first row to the last row of the part where an operation is performed,

d-1) obtaining a maximum value of any one operation value of addition selected from i-1) an operation value of addition in the relationship with an above matrix element and i-2) an operation value of addition in the relationship with a left matrix element, with i-3) an operation value of addition in the relationship with a left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-2) the operation value of addition in the relationship with the left matrix element is an operation value of addition obtained by adding the gap score (k, k is an integer) to the value of the left matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the m $\times$ 1 matrix and the 1 $\times$ n matrix, and an AND operation of 4 bits of the nucleotide that is an element of the m $\times$ 1 matrix and 4 bits of the nucleotide that is an element of the 1 $\times$ n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with any one operation value of addition from i-1) and i-2) not selected in step d-1), respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element;

(e) performing backtracking according to the indication in step d-2) from the matrix element that satisfies a predetermined score threshold value among the operation values of the matrix elements in the last column of the part where an operation is performed where the operation value is input; and

(f) providing alignment information between nucleotides of the target nucleic acid sequence and nucleotides of the oligonucleotide sequence according to the origin of the operation value of the backtracked matrix element; wherein the alignment information comprises information selected from the group consisting of a match, a mismatch, and/or a gap between nucleotides of the target nucleic acid sequence and the oligonucleotide sequence, an alignment pattern comprising the match, mismatch, and/or gap information, the number of the mismatch, and the position of the oligonucleotide sequence in aligned target nucleic acid sequence.

3. The method according to claim 1 or 2, wherein the oligonucleotide is a primer or probe.

4. The method according to claim 1 or 2, wherein m and n are natural numbers.

5. The method according to claim 1 or 2, wherein the m has a number greater than n.

6. The method according to claim 1 or 2, wherein the base comprises a degenerate base and/or universal base.

7. The method according to claim 1 or 2, wherein the oligonucleotide is an oligonucleotide represented by the following formula (I):

$$5'\text{-}X\text{-}Y\text{-}Z\text{-}3' \qquad (I)$$

wherein, X represents a portion comprising a hybridizing nucleotide sequence that is hybridized with the target nucleic acid sequence, Y represents a separation portion comprising two or more consecutive bases that do not involve in Watson-Crick base pairing, and Z represents a portion comprising a hybridizing nucleotide sequence that is hybridized with the target nucleic acid sequence.

8. The method according to claim 1 or 2, wherein the predetermined match score and mismatch score are integers.

9. The method according to claim 1 or 2, wherein when the matrix operation value is same as 4 bits of the nucleotide of the target nucleic acid sequence among the nucleotides on which the matrix operation is performed, the nucleotides on which the matrix operation is performed represent being a match, and the match value is a value expressing the match as a predetermined match score.

10. The method according to claim 1 or 2, wherein when the matrix operation value is same as 4 bits of the nucleotide of the oligonucleotide sequence among the nucleotides on which the matrix operation is performed, the nucleotides on which the matrix operation is performed represent being a match, and the match value is a value expressing the match as a predetermined match score.

11. The method according to claim 1 or 2, wherein the method further comprises that when the matrix operation value is different from 4 bits of the nucleotide of the target nucleic acid sequence among the nucleotides on which the matrix operation is performed, the nucleotides on which the matrix operation is performed represent being a mismatch, and the mismatch value is a value expressing the mismatch as a predetermined mismatch score.

12. The method according to claim 1, wherein that the sum satisfies a predetermined match threshold value represents that the sum is greater than or equal to a predetermined match threshold value, and that the sum satisfies a predetermined mismatch threshold value represents that the sum is less than or equal to a predetermined mismatch threshold value.

13. The method according to claim 1 or 2, wherein the method provides coverage of oligonucleotides for a plurality of target nucleic acid sequences.

14. The method according to claim 2, wherein the origin point value is an integer.

15. The method according to claim 2, wherein the operation is performed by a method comprising the following steps for each row in order from the first row to the last row of the part where an operation is performed:

d-1) obtaining a maximum value of i-1) an operation value of addition in the relationship with the above matrix element with i-3) an operation value of addition in the relationship with the left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the m $\times$ 1 matrix and the 1 $\times$ n

matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with i-2) the operation value of addition in the relationship with the left matrix element, respectively, in order from the first matrix element to the last matrix element of the row of the part where operation is performed, inputting the maximum value as the operation value of each matrix element of the part where an operation is performed, and indicating that the input operation value is a value originated from which matrix element among the above, the left diagonal, and the left matrix elements of the corresponding matrix element; wherein i-2) the operation value of addition in the relationship with the left matrix element is an operation value of addition obtained by adding the gap score (k, k is an integer) to the value of the left matrix element of each matrix element of the row of the part where an operation is performed.

16. The method according to claim 2, wherein the operation is performed by a method comprising the following steps for each row in order from the first row to the last row of the part where an operation is performed:

d-1) i-1) obtaining an operation value of addition by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element; wherein the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score;

i-2) obtaining an operation value of addition by adding the gap score (k, k is an integer) to the value of the above matrix element of each matrix element of the row of the part where an operation is performed;

i-3) obtaining a maximum value of the operation value of addition obtained in step i-1) and the operation value of addition obtained in step i-2) of each matrix element for the row of the part where the operation is performed, and inputting the maximum value as an operation value for each matrix element; and

d-2) obtaining the maximum value of the operation value of each matrix element input in i-3) of step d-1) with the operation value of addition obtained by adding the gap score (k) (k is an integer) to the value of the left matrix element of each matrix element of the row of the part where an operation is performed, respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element.

17. The method according to ef claim 2, wherein in step (e), satisfying a predetermined score threshold value among the operation values represents that the operation value is greater than or equal to a predetermined score threshold value.

18. The method according to claim 2, wherein the predetermined score threshold value is calculated by the following equation 1:

**Equation 1** Score threshold value = (mismatch threshold value x mismatch score) + ((length of oligonucleotide - mismatch threshold value) x match score)

Equation 1

19. The method according to claim 2, wherein (i) when the operation value is originated from the left diagonal matrix element of the matrix element, the alignment information comprises match or mismatch information of the nucleotide of the target nucleic acid sequence of the row and the nucleotide of the oligonucleotide sequence of the column corresponding to the matrix element, (ii) when the operation value is originated from the left matrix element of the matrix element, the alignment information comprises gap information between the nucleotide of the target nucleic acid sequence of the row and the nucleotide of the target nucleic acid sequence of the next row of the matrix element, and (iii) when the operation value is originated from the above matrix element of the matrix element, the alignment information comprises gap information between the nucleotide of the oligonucleotide sequence of the column and the nucleotide of the oligonucleotide sequence of the next column of the matrix element.

20. A computer readable storage medium containing instructions to configure a processor to perform a method for providing alignment information of an oligonucleotide for a target nucleic acid sequence, the method comprising:

(a) providing a target nucleic acid sequence comprising m nucleotides and an oligonucleotide sequence comprising n nucleotides; wherein the target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type,

(b) performing a matrix operation of the oligonucleotide sequence for the target nucleic acid sequence to obtain a match value or mismatch value between nucleotides of the target nucleic acid sequence and the oligonucleotide sequence; wherein the matrix operation is a multiplication of the m × 1 matrix and the 1 × n matrix, and an AND operation of 4 bits of the nucleotide that is an element of the m × 1 matrix and 4 bits of the nucleotide that is an element of the 1 × n matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score,

(c) performing a trace operation of a n × n square matrix in the m × n matrix on which the matrix operation is performed to obtain the sum of match values and/or mismatch values of diagonal elements of the n × n square matrix; and

(d) when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold value; wherein the alignment information comprises information selected from the group consisting of a match and/or mismatch between nucleotides of the target nucleic acid sequence and the oligonucleotide sequence, an alignment pattern comprising the match and/or mismatch information, the number of the mismatch, and the position of the oligonucleotide sequence in aligned target nucleic acid sequences.

21. A computer readable storage medium containing instructions to configure a processor to perform a method for providing alignment information of an oligonucleotide for a target nucleic acid sequence, the method comprising:

(a) providing a target nucleic acid sequence comprising m nucleotides and an oligonucleotide sequence comprising n nucleotides; wherein the target nucleic acid sequence is provided as a m × 1 matrix comprising m nucleotides as matrix elements, the oligonucleotide sequence is provided as a 1 × n matrix comprising n nucleotides as matrix elements, and the nucleotides are expressed in 4 bits for each base type,

(b) providing an operation table; wherein the operation table comprises a part where operation is performed and a part where a gap score is input, the part where operation is performed comprises elements ($C_{i,j}$, $1 \leq i \leq m$, $1 \leq j \leq n$) of the m×n matrix obtained by multiplying the m×1 matrix and the 1×n matrix, and the part where a gap score is input comprises an origin point ($C_{0,0}$), 0 row elements ($C_{0,j}$, $1 \leq j \leq n$), and 0 column elements ($C_{i,0}$, $1 \leq i \leq m$),

(c) inputting a gap score into the part where a gap score is input of the operation table; wherein origin point value + (j x gap score (k)) ($1 \leq j \leq n$, k is an integer) is input for each column element in the 0 row elements of the part where a gap score is input, and origin point value + (i x gap score (k)) ($1 \leq i \leq n$, k is an integer) is input for each row element in the 0 column elements of the part where a gap score is input,

(d) performing an operation and inputting an operation value into the part where an operation is performed of the operation table; wherein the operation is performed by a method comprising the following steps for each row in order from the first row to the last row of the part where an operation is performed,

d-1) obtaining a maximum value of any one operation value of addition selected from i-1) an operation value of addition in the relationship with an above matrix element and i-2) an operation value of addition in the relationship with a left matrix element, with i-3) an operation value of addition in the relationship with a left diagonal matrix element of each matrix element for the row of the part where an operation is performed, and inputting the maximum value as an operation value of each matrix element; wherein i-1) the operation value of addition in the relationship with the above matrix element is an operation value of addition obtained by adding the gap score (k, k is an integer) to the value of the above matrix element of each matrix element of the row of the part where an operation is performed, i-2) the operation value of addition in the relationship with the left matrix element is an operation value of addition obtained by adding the gap score (k, k is an integer) to the value of the left matrix element of each matrix element of the row of the part where an operation is performed, i-3) an operation value of addition in the relationship with the left diagonal matrix element is an operation value of addition obtained by performing a matrix operation of the oligonucleotide sequence for the target nucleic acid sequence for the row of the part where an operation is performed to obtain a match value or mismatch value between nucleotides of the target nucleic acid sequence and the oligonucleotide sequence for each matrix element, and adding the match value or mismatch value for each matrix element and the value of the left diagonal matrix element of each matrix element, the matrix operation is a multiplication of the $m \times 1$ matrix and the $1 \times n$ matrix, and an AND operation of 4 bits of the nucleotide that is an element of the $m \times 1$ matrix and 4 bits of the nucleotide that is an element of the $1 \times n$ matrix, when matrix operation value is the same as 4 bits of any one of the nucleotides on which the matrix operation is performed, the nucleotides on which the matrix operation is performed represent being a match and the match value is a value expressing the match as a predetermined match score, and when matrix operation value is different from 4 bits of all nucleotides on which the matrix operation is performed, the nucleotides on which the matrix operation is performed represent being a mismatch and the mismatch value is a value expressing the mismatch as a predetermined mismatch score, and

d-2) obtaining the maximum value of the operation value of each matrix element input in step d-1) with any one operation value of addition from i-1) and i-2) not selected in step d-1), respectively, in order from the first matrix element to the last matrix element of the row of the part where an operation is performed, inputting the maximum value as the operation value of each matrix element of the part where an operation is performed, and indicating that the input operation value is a value originated from which matrix element among the above, the left diagonal, and the left matrix elements of the corresponding matrix element;

(e) performing backtracking according to the indication in step d-2) from the matrix element that satisfies a predetermined score threshold value among the operation values of the matrix elements in the last column of the part where an operation is performed where the operation value is input; and

(f) providing alignment information between nucleotides of the target nucleic acid sequence and nucleotides of the oligonucleotide sequence according to the origin of the operation value of the backtracked matrix element; wherein the alignment information comprises information selected from the group consisting of a match, a mismatch, and/or a gap between nucleotides of the target nucleic acid sequence and the oligonucleotide sequence, an alignment pattern comprising the match, mismatch, and/or gap information, the number of the mismatch, and the position of the oligonucleotide sequence in aligned target nucleic acid sequence.

# Fig. 1

*100*

110 — 
| providing a target nucleic acid sequence comprising m nucleotides and an oligonucleotide sequence comprising n nucleotides |

120 — 
| performing a matrix operation of the oligonucleotide sequence for the target nucleic acid sequence to obtain a match value or mismatch value between nucleotides of the target nucleic acid sequence and the oligonucleotide sequence |

130 — 
| performing a trace operation of a n × n square matrix in the m × n matrix on which the matrix operation is performed to obtain the sum of match values and/or mismatch values of diagonal elements of the n × n square matrix |

140 — 
| when the sum satisfies a predetermined match or mismatch threshold value, providing alignment information of the nucleotides of the target nucleic acid sequence of row elements and the nucleotides of the oligonucleotide sequence of column elements corresponding to the diagonal elements of the n × n square matrix having the sum satisfying the predetermined threshold |

# Fig. 2

|   | C | A | G | C | A | G |
|---|---|---|---|---|---|---|
| T | 1 | 1 | 1 | 1 | 1 | 1 |
| C | 0 | 1 | 1 | 0 | 1 | 1 |
| A | 1 | 0 | 1 | 1 | 0 | 1 |
| G | 1 | 1 | 0 | 1 | 1 | 0 |
| C | 0 | 1 | 1 | 0 | 1 | 1 |
| C | 0 | 1 | 1 | 0 | 1 | 1 |
| A | 1 | 0 | 1 | 1 | 0 | 1 |
| G | 1 | 1 | 0 | 1 | 1 | 0 |
| C | 0 | 1 | 1 | 0 | 1 | 1 |
| T | 1 | 1 | 1 | 1 | 1 | 1 |
| G | 1 | 1 | 0 | 1 | 1 | 0 |
| C | 0 | 1 | 1 | 0 | 1 | 1 |
| A | 1 | 0 | 1 | 1 | 0 | 1 |

# Fig. 3

|   | C | A | G | C | A | G |
|---|---|---|---|---|---|---|
| T | 1 | 1 | 1 | 1 | 1 | 1 |
| C | 0 | 1 | 1 | 0 | 1 | 1 |
| A | 1 | 0 | 1 | 1 | 0 | 1 |
| G | 1 | 1 | 0 | 1 | 1 | 0 |
| C | 0 | 1 | 1 | 0 | 1 | 1 |
| C | 0 | 1 | 1 | 0 | 1 | 1 |
| A | 1 | 0 | 1 | 1 | 0 | 1 |
| G | 1 | 1 | 0 | 1 | 1 | 0 |
| C | 0 | 1 | 1 | 0 | 1 | 1 |
| T | 1 | 1 | 1 | 1 | 1 | 1 |
| G | 1 | 1 | 0 | 1 | 1 | 0 |
| C | 0 | 1 | 1 | 0 | 1 | 1 |
| A | 1 | 0 | 1 | 1 | 0 | 1 |

# Fig. 4

*200*

**210** — providing a target nucleic acid sequence comprising m nucleotides and an oligonucleotide sequence comprising n nucleotides

**220** — providing an operation table

**230** — inputting a gap score into the part where a gap score is input of the operation table

**240** — performing an operation and inputting an operation value into the part where an operation is performed of the operation table

**250** — performing backtracking according to the indication from the matrix element that satisfies a predetermined score threshold value among the operation values of the matrix elements in the last column of the part where an operation is performed where the operation value is input

**260** — providing alignment information between nucleotides of the target nucleic acid sequence and nucleotides of the oligonucleotide sequence according to the origin of the operation value of the backtracked matrix element

# Fig. 5

|   |   | C | A | G | C | A | G |
|---|---|---|---|---|---|---|---|
|   | 0 → | -5 → | -10 → | -15 → | -20 → | -25 → | -30 |
| T | 0 | 0 | 0 | 0 | 0 | 0 | 0 |
| C | 0 | 0 | 0 | 0 | 0 | 0 | 0 |
| A | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| C | 0 | 0 | 0 | 0 | 0 | 0 | 0 |
| A | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| T | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| A | 0 | 0 | 0 | 0 | 0 | 0 | 0 |

# Fig. 6

|   |   | C | A | G | C | A | G |
|---|---|---|---|---|---|---|---|
|   | 0 | -5 | -10 | -15 | -20 | -25 | -30 |
| T | 0 | -1 | -6 | -11 | -16 | -21 | -26 |
| C | 0 | 0 | 0 | 0 | 0 | 0 | 0 |
| A | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| C | 0 | 0 | 0 | 0 | 0 | 0 | 0 |
| A | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| T | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| A | 0 | 0 | 0 | 0 | 0 | 0 | 0 |

# Fig. 7

|   |   | C | A | G | C | A | G |
|---|---|---|---|---|---|---|---|
|   | 0 | -5 | -10 | -15 | -20 | -25 | -30 |
| T | 0 | -1 | -6 | -11 | -16 | -21 | -26 |
| C | 0 | 0 | 0 | 0 | 0 | 0 | 0 |
| A | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| C | 0 | 0 | 0 | 0 | 0 | 0 | 0 |
| A | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| T | 0 | 0 | 0 | 0 | 0 | 0 | 0 |
| G | 0 | 0 | 0 | 0 | 0 | 0 | 0 |
| C | 0 | 0 | 0 | 0 | 0 | 0 | 0 |
| A | 0 | 0 | 0 | 0 | 0 | 0 | 0 |

# Fig. 8

|   |   | C | A | G | C | A | G |
|---|---|---|---|---|---|---|---|
|   | 0 | -5 | -10 | -15 | -20 | -25 | -30 |
| T | 0 | -1 | -6 | -11 | -16 | -21 | -26 |

|   |   | C | A | G | C | A | G |
|---|---|---|---|---|---|---|---|
|   | 0 | -5 | -10 | -15 | -20 | -25 | -30 |
| T | 0 | -1 | -6 | -11 | -16 | -21 | -26 |

|   |   | C | A | G | C | A | G |
|---|---|---|---|---|---|---|---|
|   | 0 | -5 | -10 | -15 | -20 | -25 | -30 |
| T | 0 | -1 | -6 | -11 | -16 | -21 | -26 |

|   |   | C | A | G | C | A | G |
|---|---|---|---|---|---|---|---|
|   | 0 | -5 | -10 | -15 | -20 | -25 | -30 |
| T | 0 | -1 | -6 | -11 | -16 | -21 | -26 |

# Fig. 9

|   |   | C | A | G | C | A | G |
|---|---|---|---|---|---|---|---|
|   | 0 | -5 | -10 | -15 | -20 | -25 | -30 |
| T | 0 | -1 | -6 | -11 | -16 | -21 | -26 |
| C | 0 | 1 | -2 | -7 | -10 | -15 | -20 |
| A | 0 | -1 | 2 | -3 | -8 | -9 | -14 |
| G | 0 | -1 | -2 | 3 | -2 | -7 | -8 |
| C | 0 | 1 | -2 | -2 | 4 | -1 | -6 |
| C | 0 | 1 | 0 | -3 | -1 | 3 | -2 |
| A | 0 | -1 | 2 | -1 | -4 | 0 | 2 |
| G | 0 | -1 | -2 | 3 | -2 | -5 | 1 |
| C | 0 | 1 | -2 | -2 | 4 | -1 | -4 |
| T | 0 | -1 | 0 | -3 | -1 | 3 | -2 |
| G | 0 | -1 | -2 | 1 | -4 | -2 | 4 |
| C | 0 | 1 | -2 | -3 | 2 | -3 | -1 |
| A | 0 | -1 | 2 | -3 | -3 | 3 | -2 |

# Fig. 10

|  |  | C | A | G | C | A | G |
|---|---|---|---|---|---|---|---|
|  | 0 | -5 | -10 | -15 | -20 | -25 | -30 |
| T | 0 | -1 | -6 | -11 | -16 | -21 | -26 |
| C | 0 | 1 | -2 | -7 | -10 | -15 | -20 |
| A | 0 | -1 | 2 | -3 | -8 | -9 | -14 |
| G | 0 | -1 | -2 | 3 | -2 | -7 | -8 |
| C | 0 | 0 | 1 | -2 | 4 | -1 | -6 |
| C | 0 | 1 | 0 | -3 | -1 | 3 | -2 |
| A | 0 | -1 | 2 | -1 | -4 | 0 | 2 |
| G | 0 | -1 | -2 | 3 | -2 | -5 | 1 |
| C | 0 | 1 | -2 | -2 | 4 | -1 | -4 |
| T | 0 | -1 | 0 | -3 | -1 | 3 | -2 |
| G | 0 | -1 | -2 | 1 | -4 | -2 | 4 |
| C | 0 | 1 | -2 | -3 | 2 | -3 | -1 |
| A | 0 | -1 | 2 | -3 | -3 | 3 | -2 |

# EP 4 752 885 A1

## INTERNATIONAL SEARCH REPORT

| International application No. |
| --- |
| **PCT/KR2024/010509** |

### A. CLASSIFICATION OF SUBJECT MATTER

**G16B 30/10**(2019.01)i; **C12Q 1/6811**(2018.01)i; **G16B 50/00**(2019.01)i

According to International Patent Classification (IPC) or to both national classification and IPC

### B. FIELDS SEARCHED

Minimum documentation searched (classification system followed by classification symbols)

G16B 30/10(2019.01); C12Q 1/6869(2018.01); G16B 30/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 올리고뉴클레오타이드(oligonucleotide), 얼라인먼트(alignment), 행렬(matrix), 매치(match), 미스매치(mismatch), 갭(gap), 역추적(backtracking)

### C. DOCUMENTS CONSIDERED TO BE RELEVANT

| Category* | Citation of document, with indication, where appropriate, of the relevant passages | Relevant to claim No. |
| --- | --- | --- |
| A | SETYORINI et al. The implementation of bit-parallelism for DNA sequence alignment. Journal of Physics: Conference Series. May 2017, vol. 835, no. 1, article id. 012004, pp. 1-9. See abstract; pages 2-3; and figures 2-3. | 1-21 |
| A | US 10991453 B2 (LIFE TECHNOLOGIES CORPORATION) 27 April 2021 (2021-04-27) See columns 27 and 31; and claims 1 and 8. | 1-21 |
| A | LAKHANI, Jyoti et al. MPSAGA: a matrix-based pair-wise sequence alignment algorithm for global alignment with position based sequence representation. Sadhana. 29 June 2019 (online publication date), vol. 44, article no. 171, pp. 1-17. See entire document. | 1-21 |
| A | CHAURASIA, Rajashree et al. On the statistical significance of pairwise global alignments of nucleotide sequences. JP Journal of Biostatistics. 20 March 2023 (online publication date), vol. 23, no. 1, pp. 51-76. See entire document. | 1-21 |

☑ Further documents are listed in the continuation of Box C.  ☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **31 October 2024** | **31 October 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2024/010509**

**C.    DOCUMENTS CONSIDERED TO BE RELEVANT**

| Category* | Citation of document, with indication, where appropriate, of the relevant passages | Relevant to claim No. |
|---|---|---|
| A | SALIMAN, Nur Farah Ain et al. Systolic processing element based on the viterbi algorithm for DNA sequence alignment. 2013 IEEE Conference on Systems, Process & Control (ICSPC2013). 13-15 December 2013, pp. 268-274. DOI: 10.1109/SPC.2013.6735145.<br>      See entire document. | 1-21 |

Form PCT/ISA/210 (second sheet) (July 2022)

## INTERNATIONAL SEARCH REPORT

International application No.

**PCT/KR2024/010509**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT
### Information on patent family members

International application No.

**PCT/KR2024/010509**

| Patent document cited in search report | | | Publication date (day/month/year) | Patent family member(s) | | | Publication date (day/month/year) |
|---|---|---|---|---|---|---|---|
| US | 10991453 | B2 | 27 April 2021 | US | 2012-0197623 | A1 | 02 August 2012 |
| | | | | US | 2014-0220558 | A1 | 07 August 2014 |
| | | | | US | 2018-0196915 | A1 | 12 July 2018 |
| | | | | US | 8594951 | B2 | 26 November 2013 |
| | | | | US | 9824180 | B2 | 21 November 2017 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20230096963
- US 4683195 A, Mullis
- US 4683202 A
- US 4800159 A
- WO 2017209575 A
- WO 1999014226 A
- WO 2005021570 A
- WO 2006095981 A
- WO 2012096523 A
- US 8440406 B
- US 7422850 B
- WO 2013123552 A
- WO 2014124290 A
- WO 2011028041 A
- US 8092997 B

**Non-patent literature cited in the description**

- **SAIKI et al.** *Science*, 1985, vol. 230, 1350-1354
- PCR. **MCPHERSON** ; **MOLLER**. BIOS Scientific Publishers. Springer-Verlag, 2000
- **ALTSCHUL et al.** *J. Mol. Biol.*, 1990, vol. 215, 403-10
- **M. EGHOLM et al.** *Nature*, 1993, vol. 365, 566-568
- **WOJCIECH RYCHLIK**. OLIGO 7 Primer Analysis Software. *Methods Mol Biol*, 2007, vol. 402, 35-60